(19) Europäisches Patentamt / European Patent Office / Office européen des brevets

(11) **EP 3 543 358 A1**

(12) **EUROPEAN PATENT APPLICATION**

(43) Date of publication:
**25.09.2019 Bulletin 2019/39**

(51) Int Cl.:
***C12Q 1/6883*** (2018.01)

(21) Application number: **18382194.1**

(22) Date of filing: **22.03.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Institut d'Investigació Biomèdica de Bellvitge (IDIBELL)**
 **08908 Hospitalet de Llobregat (ES)**
• **Fundació Puigvert**
 **08025 Barcelona (ES)**

(72) Inventors:
• **LARRIBA BARTOLOMÉ, Sara**
 **08028 Barcelona (ES)**
• **BASSAS ARNAU, Lluís**
 **08911 Badalona (Barcelona) (ES)**

(74) Representative: **Herrero & Asociados, S.L.**
 **Cedaceros, 1**
 **28014 Madrid (ES)**

(54) **METHODS AND MARKERS FOR AZOOSPERMIA CHARACTERISATION**

(57) The present invention belongs to the field of male infertility, and particularly to methods, markers and kits based on miRNAs, useful for determining the origin of azoospermia and for predicting the presence of sperm in testes of a subject with azoospermia, and particularly in subjects with non-obstructive azoospermia.

**EP 3 543 358 A1**

**Description**

Field of the Invention

The present invention refers to the field of male infertility and, in particular, to methods, markers and kits for determining the origin of azoospermia and the presence or absence of sperm in the testes of azoospermic subjects.

Background of the Invention

Approximately 4% of men worldwide suffer from infertility. In a high percentage of those men, the aetiology of infertility is closely related to alterations of the classic parameters of basic semen analysis, such as the concentration, motility and/or morphology of the spermatozoa. Routine biochemical markers in semen, such as acid phosphatase, citric acid and zinc (prostate markers), fructose (seminal vesicles) and alpha-glucosidase (epididymis), are used to diagnose possible acquired and congenital obstructions or to identify functional abnormalities of the genital glands such as those that occur after infection or inflammation. Though still useful, these biochemical markers have limited clinical utility for classifying the origin of the sperm defects in semen, and the spermatogenic reserve of the testes in those men with a total absence of sperm in the ejaculate or azoospermia, a particularly challenging condition which accounts for more than 10% cases of male infertility.

In the last decade multiple efforts have been made in order to develop specific biomarkers to contribute in the diagnosis and prognosis of male infertility and to increase the success of reproductive treatments. At present, assessment of azoospermic patients is based on medical history, physical findings, hormone analysis, karyotype and a limited number of genetic tests. However, in some cases the diagnosis remains elusive and testicular biopsy is necessary. Furthermore, even in suspected non-obstructive azoospermia, currently available markers, such as elevated levels of Follicle Stimulating Hormone (FSH) in blood or diminished testicular volume among others, are unsatisfactory, and thus testis biopsy is also mandatory to identify men with some residual spermatogenesis. For these reasons, a non-invasive screening test that could identify those individuals with real chances of positive sperm recovery on the biopsy would be welcomed.

MiRNAs are small non-coding RNAs (19-23 nucleotides) that negatively modulate gene expression at the post-transcriptional level, affecting mRNA stability and translation by interacting with complementary sites on the 3'UTR of the target mRNAs. Studies have shown that miRNAs play critical roles in a variety of biological processes such as: cell proliferation, differentiation, apoptosis and carcinogenesis. MiRNAs actively participate in diverse aspects of vertebrate differentiation and development and, specifically, in testis differentiation in the embryo, male germline development and sperm production. MiRNAs are not only present in cells but also in extracellular milieu especially in different biofluids, such as blood plasma, saliva, tears, urine, breast milk, colostrum, peritoneal fluid, cerebrospinal fluid, bronchial lavage and seminal fluid. Such extracellular miRNAs are presented either bound with protein complexes or contained in extracellular vesicles (EVs) such as the exosomes. This attribute allows miRNAs to be remarkably resistant to the presence of high RNAse activity in the extracellular environment, making them suitable markers for clinical applications, such as diagnosis of different diseases and conditions. MiRNAs have already been described as markers for the diagnosis of prostate cancer (e.g. WO 2011/080315 A1) or melanoma (e.g. EP 2598657 B1). Moreover, they have been described as useful markers for diagnosis of different conditions related to male infertility, for example, as useful markers for the detection of spermatogenic failure in testicular samples (e.g. CN 106337088 A1).

However, even though markers for the detection of spermatogenic failure have already been described, there is still pending in the state of the art a miRNA or set of miRNAs which help predicting the presence of residual spermatogenesis in testes of subjects with azoospermia in a non-invasive manner. This information would be useful in the clinics, providing realistic information about the chances of sperm retrieval from the testes in order to avoid unnecessary biopsies. Interestingly, the authors of the present invention have developed miRNA-based methods, markers and kits able to determine the origin of azoospermia and to predict the presence of residual spermatogenesis in testes using seminal samples.

Summary of the Invention

In a first aspect, the present invention refers to an *in vitro* method for predicting the presence of sperm in the testes of a subject, comprising the analysis of the expression profile of a predetermined set of miRNAs in a biological sample taken from said subject, wherein said subject has non-obstructive azoospermia and wherein said predetermined set of miRNAs comprises miR-539-5p and miR-941.

In a second aspect, the present invention refers to an *in vitro* method for determining the origin of azoospermia, comprising the analysis of the expression profile of a predetermined set of miRNAs in a biological sample taken from an azoospermic subject, wherein said predetermined set of miRNAs comprises one or more miRNAs selected from the group consisting of miR-539-5p, miR-941, miR-31-5p, miR-205-5p, miR-182-3p, miR-192-3p, miR-19a-3p, miR-200a-3p, miR-23b-5p, miR-363-3p, miR-451a, miR-934 and combination thereof.

In a third aspect, the present invention refers to a set of miRNAs for use in the prediction of the presence of sperm in the testes of a subject with non-obstructive azoospermia, wherein the set of miRNAs comprises miR-539-5p and miR-941.

In a fourth aspect, the present invention refers to kits to carry out the method according to the first aspect or the second aspect of the invention.

In a fifth aspect, the present invention refers to the use of the kits of the fourth aspect of the invention, for determining the origin of azoospermia or for predicting the presence of sperm in the testes of an azoospermic subject or of a non-obstructive azoospermic subject.

Other objects, features, advantages and aspects of the present application will become apparent to those skilled in the art from the following description and appended claims.

Brief Description of the Drawings

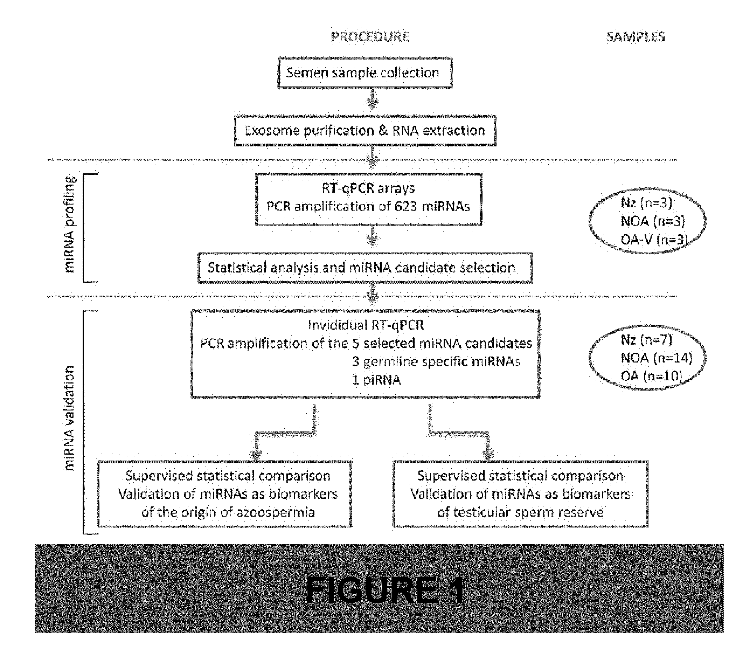

**Figure 1.-** Flow chart representing the two stages design study. The number of miRNAs and individuals analysed in each work-procedure stage are depicted.

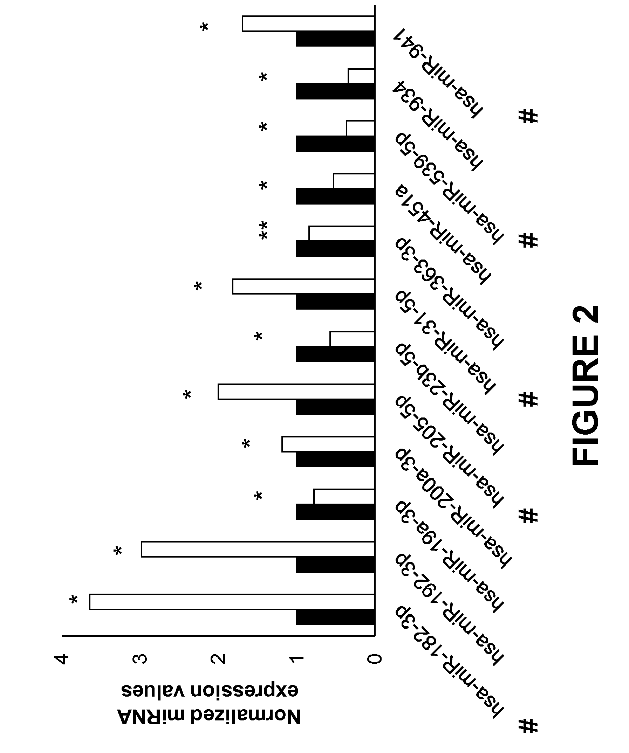

**Figure 2.-** Differential abundance profile of exosomal miRNAs by RT-qPCR arrays from semen samples of successfully vasectomized obstructive azoospermic men (black bars) and non-obstructive azoospermic men (white bars). Normalized expression levels relative to the mean of the 50 stable miRNAs are shown. Significant differences between groups are indicated: *$p$-value<0.05; **$p$-value<0.01 (Student T-test). Those miRNAs selected for validation are indicated by a # symbol.

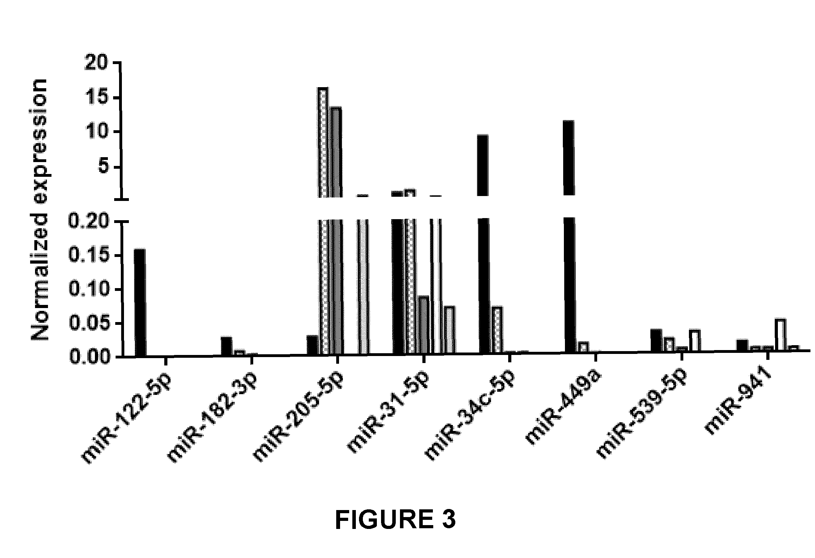

**Figure 3.-** Tissue expression profile of 8 miRNA candidates determined by RT-qPCR in several reproductive organs such as testes (black), epididymis (dotted) and prostate (dark grey), as well as in exosomes from seminal plasma (light grey) and lymphocytes (white). Expression levels relative to miR-30d-5p and miR-30e-3p are shown.

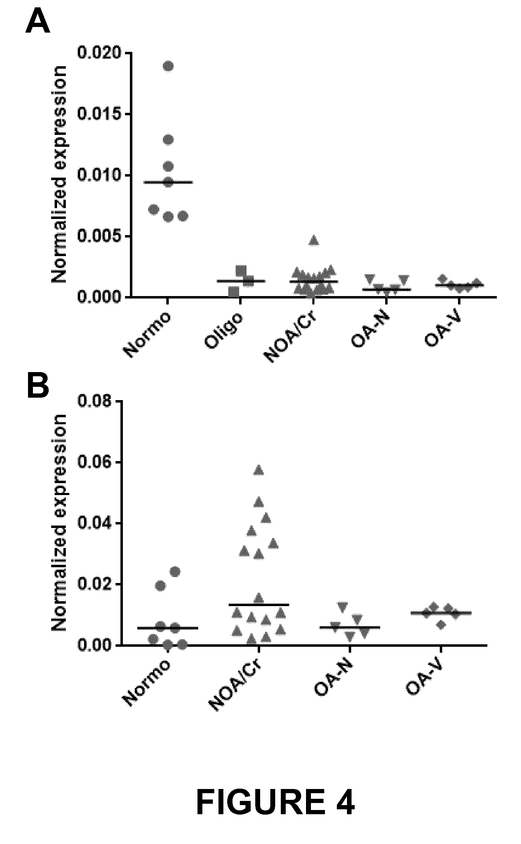

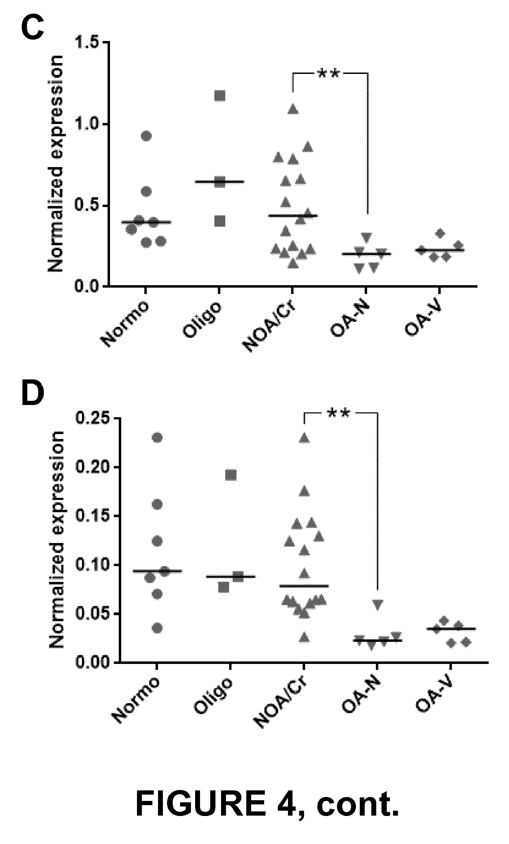

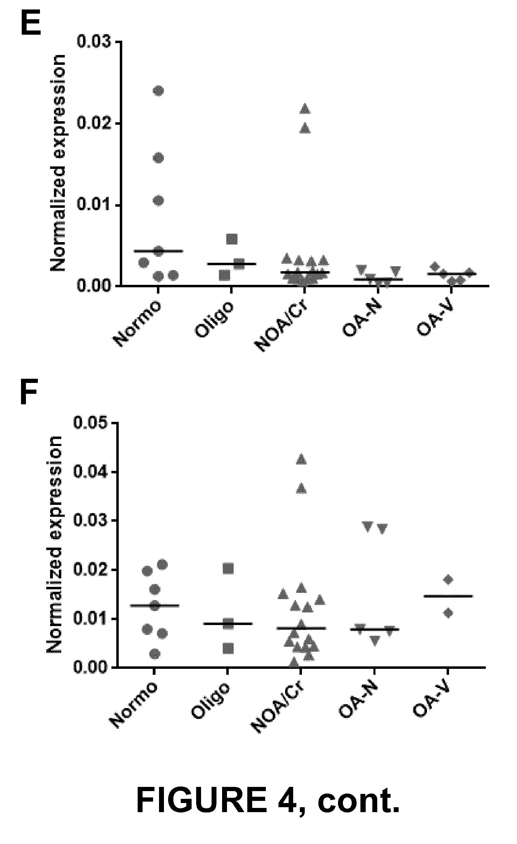

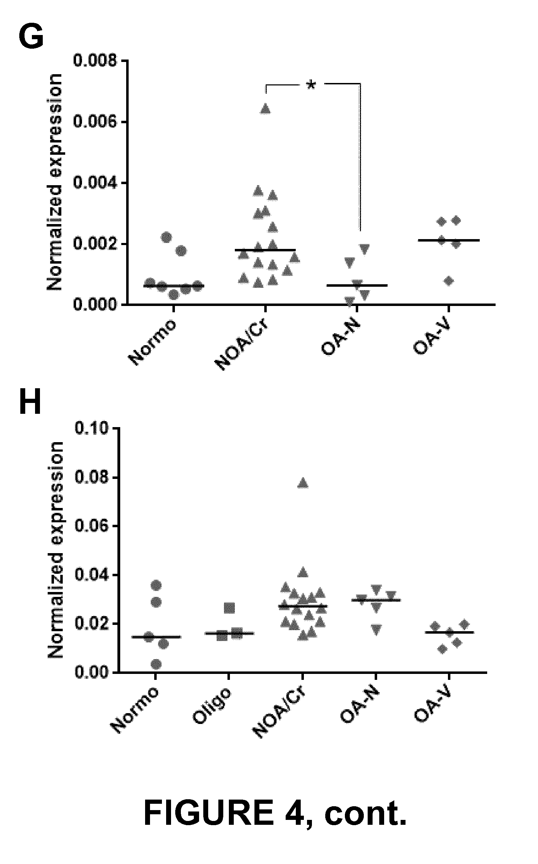

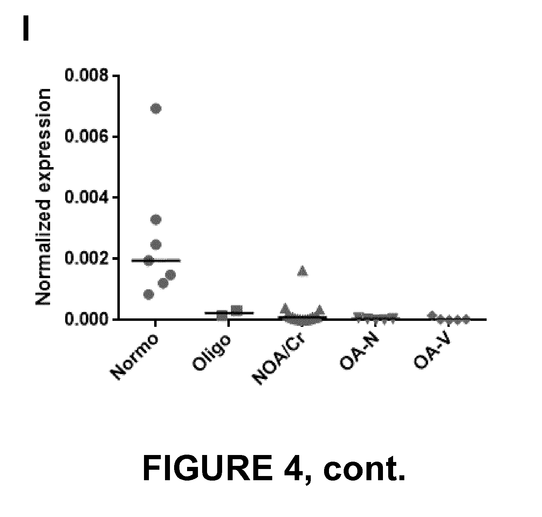

**Figure 4.-** Expression profiling, at the validating stage, of the miRNAs/piRNA in exosomes from semen of normozoospermic (Normo), oligozoospermic (Oligo), non-obstructive azoospermia or cryptozoospermia (NOA/Cr), successfully vasectomized obstructive azoospermic men (OA-V), and individuals presenting a pathological naturally occurring-obstruction in the genital tract (OA-N). The miRNAs/piRNA analysed were miR-122 (panel A), miR-182-3p (panel B), miR-205-5p (panel C), miR-31-5p (panel D), miR-34-5p (panel E), miR-449a (panel F), miR-539-5p (panel G), miR-941 (panel H) and piR-58527 (panel I). The horizontal bar displays the median cellular expression level. Significant differences between OA-N and NOA/Cr groups are indicated: *$p<0.05$; **$p<0.01$ (Mann Whitney U test). Other significant differences are indicated in the examples.

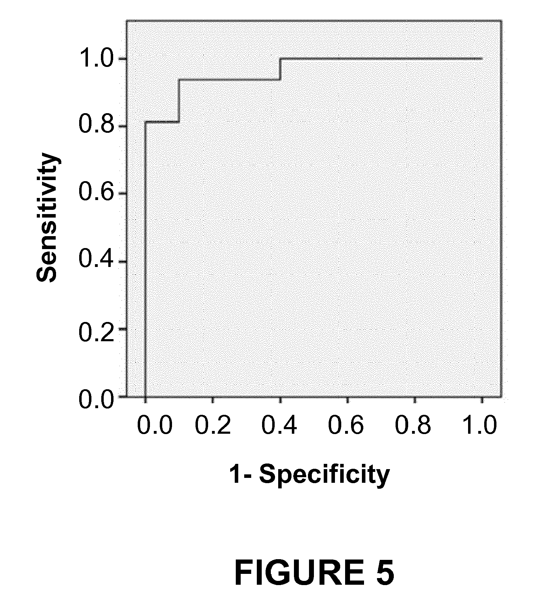

**Figure 5.-** Receiver operating characteristic (ROC) curve of miR-31-5p for predictive classification of azoospermic samples into NOA and OA sub-phenotypes at the validation stage.

Detailed Description of the Invention

It must be noted that as used in the present application, the singular forms, e.g. "a", "an" and "the", include their correspondent plurals unless the context clearly dictates otherwise. Unless defined otherwise, all technical and scientific terms used herein have the same meaning as commonly understood by one of ordinary skill in the art to which this invention belongs.

To facilitate understanding and clarify the meaning of specific terms in the context of the present invention, the following definitions and particular and preferred embodiments thereof, applicable to all the embodiments of the different aspects of the present invention, are provided:

In the context of the present invention, the term "azoospermia" (abbreviated as AZO hereinafter) refers to no sperm (absence of sperm) in semen. Sperm refers to mature, motile spermatozoa.

"Obstructive azoospermia" (abbreviated as OA hereinafter) refers to no sperm in semen even though the subject has conserved spermatogenesis. As shown in the examples, in the experimental studies carried out by the inventors to develop the present invention, OA comprises naturally-occuring obstructive azoospermia (OA-N) and obstructive azoospermia due to a successful vasectomy (OA-V). It is important to remember the aim of the present invention, which is to find out the origin of the absence of sperm in the ejaculate of a congenital azoospermic subject (i.e. a vasectomised subject would not be in this population since the origin of his azoospermia is known for this subject who is surgically azoospermic for permanent contraception by his own will) as well as to predict whether the congenital azoospermic patient has or not residual spermatogenesis. Thus, in a preferred embodiment of the present invention, obstructive azoospermia refers to naturally-occurring obstructive azoospermia, i.e. it excludes vasectomised subjects. "Non-obstructive azoospermia" (abbreviated as NO-A or NOA hereinafter) refers to no sperm in semen because of sperm production failure or spermatogenic failure, which can result from different reasons, e.g. hormonal problems and other clinical factors (varicocele, infection, immunologic factors, anatomic malformation, or chemical insults) or genetic causes (chromosomal aberration or a Y-chromosome microdeletion).

As used herein, the expression "the presence of residual spermatogenesis" refers to the presence of sperm in the testes. Thus, the expressions "the presence of residual spermatogenesis" and "the presence of sperm in the testes" are used interchangeably in the context of the present invention.

The terms "microRNA", "miR" or "miRNA" are used interchangeably for describing a class of non-coding RNA molecules of 19-23 nucleotides that exist in a variety of organisms, including mammals, and are conserved among species in evolution. A well established repository of validated miRNAs is the miRBase. The miRBase (www.mirbase.org) is a searchable database of published miRNA sequences and annotation. Each entry in the miRBase Sequence database represents a predicted hairpin portion of a miRNA transcript (termed mir in the database), with information on the location and sequence of the mature miRNA sequence (termed miR). Both hairpin and mature sequences are available for searching and browsing. All sequence and annotation data are also available for downloading.

The "(predetermined) set of miRNAs" is understood in the present invention as a fixed defined set of miRNAs which is able to differentiate between two different conditions (e.g. a condition 1 and another condition 2). The (predetermined) set of miRNAs may comprise a single miRNA or a collection of a plurality of miRNAs. The plurality of miRNAs is at least 2 miRNAs, preferably at least 2, 3, 4, 5, 6, 7, 8, 9, 10, 11 or 12, preferably 2, 3, 4, 5, 6 or 7. In a preferred embodiment, condition 1 is obstructive azoospermia (OA) and condition 2 is non-obstructive azoospermia (NOA). In another preferred embodiment, condition 1 is non-obstructive azoospermia with residual spermatogenesis (abbreviated as NOA_Sp+ hereinafter) and condition 2 is non-obstructive azoospermia without residual spermatogenesis (abbreviated as NOA_Sp- hereinafter).

The term "miRNA-(expression) profile" as used in the context of the present invention, represents the expression level of a single miRNA or a collection of expression levels of a plurality of miRNAs, therefore it is a quantitative measure of individual miRNA expression levels. Hereby, each miRNA is represented by a numerical value. The higher the value of an individual miRNA the higher is the expression level of this miRNA, and the lower the value of an individual miRNA, the lower is the expression level of said miRNA.

A miRNA-profile is obtained from the RNA of a biological sample (obtained/extracted from a biological sample). As a starting material for analysis, RNA or total RNA or any fraction thereof can be used. Preferably the starting material is total RNA that is reverse transcribed. The miRNA expression profile may be determined by any convenient means, e. g. nucleic acid hybridization (e.g. to a microarray, bead-based methods, paper-based methods), nucleic acid amplification (e.g. polymerase chain reaction (PCR), quantitative PCR (qPCR), reverse transcription-quantitative PCR (RT-qPCR), high-throughput RT-qPCR), next generation sequencing (e.g. ABI SOLID, Illumina Genome Analyzer, 35 Rochel454 GS FLX), flow cytometry (e.g. LUMINEX, Firefly Bioworks) and the like, that allow the analysis of differential miRNA expression levels between samples. The sample material measured by the aforementioned means may be total RNA, labeled total RNA, amplified total RNA, cDNA, labeled cDNA, amplified cDNA, miRNA, labeled miRNA, amplified miRNA or any derivatives that may be generated from the aforementioned RNA/DNA species. In a preferred embodiment, the miRNA expression profile is determined by RT-qPCR.

A "biological sample" in terms of the present invention means a sample of biological fluid. In a preferred embodiment, the biological fluid is semen or seminal plasma, and more preferably seminal plasma (abbreviated as SP hereinafter). A biological sample may be provided by taking a sample from a subject, but can also be provided by using a previously taken sample.

Semen, a complex fluid composed of cells and SP, can be accessed with relative ease. Several studies have evidenced that SP contains high concentrations of EVs, which are morphologically and molecularly consistent with exosomes, which originate from multiple cellular sources in the male reproductive tract: namely prostate (prostasomes), epididymis (epididymosomes), seminal vesicles and the testes. Exosomes are cell-derived EVs present in various biological fluids. It has been calculated that in mammals, each ejaculate contains trillions of exosomes, characterized by a high content in cholesterol and sphingomyelin, and a very complex protein composition. The exosomes contain coding and non-coding RNAs that can be transferred to recipient cells to modulate their function, thus mediating paracrine signalling. In particular, exosomes in semen contain a population of small non-coding RNAs (from 20 to 100 nucleotides), including microRNAs (miRNAs) (21%), and several other RNAs such as piRNAs, y RNAs, rRNAs and tRNAs. Semen exosomes show a unique profile of miRNAs that greatly differs from that of other biological fluids.

In a preferred embodiment according to any of the embodiments of the present invention, the biological sample comprises or consists of EVs isolated from the biological sample, preferably said EVs are exosomes. Thus, in a more preferred embodiment, the biological sample comprises or consists of isolated EVs, preferably isolated exosomes, from semen or SP, and even more preferably isolated exosomes from SP (SP exosomes). In another preferred embodiment, the starting material for determining the miRNA expression profile is total RNA extracted from SP exosomes.

As used herein, the term "subject" refers to a member of the class Mammalia, including, without limitation, humans and non-human primates such as chimpazees and other apes and monkey species; farm animal such as cattle, sheep, pigs, goats and horses; domestic mammals such as dogs and cats; laboratory animals incluiding rodents such as mice, rats and guinea pigs, and the like. The subject is a male. The subject is preferably a human, i.e. a human male.

The sensitivity of any given screening test is the proportion of individuals with the condition who are correctly identified or diagnosed by the test, e.g. the sensitivity is 100%, if all individuals with a given condition have a positive test. The specificity of a given screening test is the proportion of individuals without the condition who are correctly identified or diagnosed by the test, e.g. the specificity is 100%, if all individuals without the condition have a negative test result. Thus, the sensitivity is defined as the (number of true-positive test results) / (number of true-positive + number of false-negative test results). The specificity is defined as (number of true-negative results) / (number of true-negative + number of false-positive results). The specificity of the method according to the invention is preferably from 70% to 100%, such as from 75% to 100%, more preferably 80% to 100%, more preferably 90% to 100%. Thus, in one embodiment of the present invention the specificity is 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. The sensitivity of the method according to the invention is preferably from 70% to 100%, such as from 75% to 100%, more preferably 80% to 100%, more preferably 90% to 100%. Thus, in one embodiment of the present invention the sensitivity is 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%.

The present invention is focused on the evaluation and assessment of miRNA expression profiles in order to define a specific set of miRNAs useful for predicting the presence of sperm in testes of subjects with azoospermia, in particular subjects with non-obstructive azoospermia. The set of miRNA is also useful for determining the origin of azoospermia, i.e. discriminating between obstructive azoospermia (having conserved spermatogenesis) and non-obstructive azoospermia. The expression profile of these miRNAs could be used to decide the most convenient way to achieve the obtaining of sperm for assisted fecundation (e.g. testes biopsy, semen donor), avoiding unnecessary testicular biopsies in those cases in which no sperm is predicted to be in the testes.

As shown in the examples below, it was found that the miRNA expression profile of a predetermined set of miRNAs can be efficiently used as a predictive tool for the presence of sperm in testes of azoospermic subjects, in particular of subjects with non-obstructive azoospermia.

Thus, in a **first aspect**, the present invention refers to an *in vitro* method for predicting the presence of sperm in the testes of a subject, comprising the analysis of the expression profile of a predetermined set of miRNAs in a biological sample taken from said subject, wherein the subject has non-obstructive azoospermia and wherein said predetermined set of miRNAs comprises miR-539-5p and miR-941. This method allows predicting the presence of spermatozoa in testes of non-obstructive azoospermic subjects (i.e. subjects who have been diagnosed with non-obstructive azoospermia), and therefore allows predicting the presence of sperm in a testicular biopsy. Thus, a testicular biopsy would only be made in those subjects wherein the presence has been predicted, avoiding unnecessary biopsies. As shown in Example 4, 100% sensitivity and 100% specificity for predicting residual spermatogenesis, i.e. presence of the spermatozoa in the testes, in subjects with NO-A were observed when analysing the expression level of miR-539-5p and miR-941. Thus, in a particular embodiment, the predetermined set of miRNAs described in the previous paragraphs consists of miR-539-5p and miR-941. In a preferred embodiment according to any one of the preceding embodiments, miR-539-5p is hsa-miR-539-5p (SEQ ID NO 1) and miR-941 is hsa-miR-941 (SEQ ID NO 2).

As shown in Example 4, true positive and negative rates for predicting residual spermatogenesis in the testes of 100% were also obtained when miR-539-5p and miR-941 were analysed together with the value of FSH in a sample of blood. Thus, in a preferred embodiment according to any one of the embodiments described above, the expression profile of the predetermined set of miRNAs is analysed in combination with the level of FSH in blood. More preferably, the analysis is carried out by using a multivariate binary logistic regression, e.g. using formula I (as shown in Example 4).

As shown in Example 3, it was found that a predetermined set of miRNAs was differentially expressed between azoospermic subjects with NOA and OA (see Figure 2). Thus, said predetermined set of miRNAs has the potential to be efficiently used for determining the origin of azoospermia, i.e. for discriminating between obstructive azoospermia and non-obstructive azoospermia. As shown in Figure 2, the differentially expressed miRNAs are hsa-miR-539-5p (SEQ ID NO 1), hsa-miR-941 (SEQ ID NO 2), hsa-miR-31-5p (SEQ ID NO 3), hsa-miR-205-5p (SEQ ID NO 4), hsa-miR-182-3p (SEQ ID NO 5), hsa-miR-192-3p (SEQ ID NO 6), hsa-miR-19a-3p (SEQ ID NO 7), hsa-miR-200a-3p (SEQ ID NO 8), hsa-miR-23b-5p (SEQ ID NO 9), hsa-miR-363-3p (SEQ ID NO 10), hsa-miR-451a (SEQ ID NO 11) and hsa-miR-934 (SEQ ID NO 12). In particular, miR-539-5p, miR-19a-3p, miR-23b-5p, miR-363-3p, miR-451a and miR-934 were downregulated in NOA, whereas miR-941, miR-31-5p, miR-205-5p, miR-182-3p, miR-192-3p and miR-200a-3p were downregulated in OA.

In the method according to the first aspect of the invention, the subject is a non-obstructive azoospermic subject. Such identification as a non-obstructive azoospermic subject can be made by different means. Advantageously, it can be done by determining the miRNA expression level of any of the miRNA mentioned in the previous paragraph in the same biological sample (e.g. the sample used for predicting the presence of residual spermatogenesis). Thus, in a particular embodiment of the first aspect of the invention, the azoospermic subject in which the presence of sperm in the testes is going to be predicted, is identified as non-obstructive azoospermic subject by the analysis of the expression profile of a predetermined set of miRNAs in a biological sample taken from said subject, wherein said predetermined set of miRNAs comprises one or more (e.g. all) miRNA(s) selected from the group consisting of miR-539-5p, miR-941, miR-31-5p, miR-205-5p, miR-182-3p, miR-192-3p, miR-19a-3p, miR-200a-3p, miR-23b-5p, miR-363-3p, miR-451a, miR-934 and combinations thereof.

As shown in Example 3, miR-31-5p, miR-205-5p and miR-539-5p are differentially expressed between OA-N and NOA. In particular, these miRNAs are significantly downregulated in OA-N in comparison to NOA (see Figure 4). Moreover, they result in good predictive accuracy (area under the curve (AUC)>0.84) after ROC analyses (miR-205-5p (AUC: 0.886, p=0.012); miR-31-5p (AUC: 0.957; p=0.003); miR-539-5p (AUC: 0.843; p=0.026)). Thus, in a preferred embodiment according to previous paragraph, the predetermined set of miRNA comprises miR-31-5p or miR-205-5p or miR-539-5p or combinations thereof, and more preferably it comprises or consists of miR-31-5p and/or miR-205-5p, and even more preferably it comprises or consists of miR-31-5p. As shown in Example 3, the analysis of miR-31-5p results in a very high sensitivity and specificity ($\geq$ 90%) when analysed using a multivariate regression analysis. This confirmed that miR-31-5p expression values in semen samples could be useful to predict the presence of a conserved spermatogenesis (OA) in the testes. If the subject is OA, then there is presence of sperm in the testes (i.e. it would not be necessary to carry out the analysis of miR-539-5p and miR-94) and therefore a testicular biopsy would be successful in the retrieval of sperm. If the subject is NOA, then, the *in vitro* method can continue with the analysis of the expression profile of miR-539-5p and miR-941 for predicting the presence of residual spermatogenesis in the testes.

As shown in Example 3, an increased value of sensitivity and specificity was obtained when miR-31-5p was analysed together with the value of FSH in a sample of blood. Thus, in a preferred embodiment according to any one of the embodiments described in the previous two paragraphs, the expression profile of the predetermined set of miRNAs is analysed in combination with the level of FSH in blood. More preferably, the analysis is carried out by using a multivariate binary logistic regression, e.g. using formula I (as shown in Example 3).

In a particular embodiment of the first aspect of the invention the *in vitro* method of the invention, comprises the steps:

a) determining the expression profile of a predetermined set of miRNAs comprising miR-539-5p and miR-941, in a biological sample taken from a subject diagnosed with non-obstructive azoospermia; and
b) comparing said expression profile to a reference expression profile, wherein the comparison of said determined expression profile to said reference expression profile allows for identifying said subject as having sperm in the testes.

In a particular embodiment according to the previous paragraph, the *in vitro* method comprising the following steps before step a):

a') determining the expression profile of a predetermined set of miRNAs comprising one or more of the following miRNAs: miR-182-3p, miR-192-3p, miR-19a-3p, miR-200a-3p, miR-205-5p, miR-23b-5p, miR-31-5p, miR-363-3p, miR-451a, miR539-5p, miR-941 and combinations thereof in a biological sample taken from a subject with azoospermia;
a") comparing said expression profile to a reference expression profile, wherein the comparison of said determined expression profile to said reference expression profile allows for identifying said subject as having non-obstructive azoospermia or obstructive azoospermia.

In a particular embodiment according to any one of the two preceding paragraphs, in a') the predetermined set of miRNA comprises miR-31-5p or miR-205-5p or miR-539-5p or combinations thereof, preferably the predetermined set of miRNA comprises or consists of miR-31-5p and/or miR-205-5p, and even more preferably comprises or consists of miR-31-5p.

In a particular embodiment according to any one of the three preceding paragraphs, step a') and/or step a) further comprises the determination of the FSH value in a sample of blood of said subject, and step a") and/or step b) further comprising comparing said determined FSH value to a reference FSH value, wherein the comparison of said determined FSH value to said reference FSH value, together with the comparison of the expression profile of the miRNAs, allows for identifying said subject as suffering from non-obstructive azoospermia or obstructive azoospermia.

The reference expression profile might be a reference value, a cut-off value, a threshold or the expression profile of a control sample. Preferably, if a control sample is used as reference, a positive control and/or a negative control will be used.

In a particular embodiment, in step b), the reference expression profile comprises the expression profile of the predetermined set of miRNAs in a biological sample of a subject with NOA_Sp+ (positive control).

In a particular embodiment, in step a"), the reference expression profile comprises the expression profile of the predetermined set of miRNAs in a biological sample of a NOA, OA or Nz subject, preferably NOA or Nz (See Figures 2 and 4, and Tables 4 and 5).

As indicated above, the authors of the present invention identified 12 putative markers of the origin of azoospermia (i.e. for discriminating between OA and NOA). Thus, a **second aspect** of the invention refers to an *in vitro* method for determining the origin of azoospermia (i.e. for identifying an azoospermic subject as a subject with non-obstructive azoospermia or with obstructive azoospermia), comprising the analysis of the expression profile of a predetermined set of miRNAs in a biological sample taken from an azoospermic subject, wherein said predetermined set of miRNAs comprises one or more (e.g. all) miRNAs selected from the group consisting of miR-539-5p, miR-941, miR-31-5p, miR-205-5p, miR-182-3p, miR-192-3p, miR-19a-3p, miR-200a-3p, miR-23b-5p, miR-363-3p, miR-451a, miR-934 and combinations thereof. Preferably, as explained in the first aspect of the invention, the predetermined set of miRNA comprises miR-31-5p or miR-205-5p or miR-539-5p or combinations thereof. More preferably the predetermined set comprises or consists of miR-31-5p and/or miR-205-5p, and even more preferably the predetermined set comprises or consists of miR-31-5p.

In another preferred embodiment according to any one of the embodiments of the second aspect of the invention, the method further comprises the determination of the FSH value in a sample of blood taken from said azoospermic subject. This FSH analysis, as shown in Example 3, improves the sensitivity and the specificity when combined with the analysis of miR-31-5p.

Interestingly, the authors of the invention have seen that the analysis of miR-31-5p, miR-941 and FSH value allows for predicting the presence of sperm in the testes of an azoospermic subject with 100% sensitivity and 100% specificity. Furthermore, the analysis of miR-31-5p and miR-941 is useful for predicting if an azoospermic subject is an azoospermic subject with positive testicular sperm extraction value (TESE+, i.e. OA and NOA_Sp+, presence of sperm in the testes) or an azoospermic subject with negative testicular sperm extraction value (TESE-, i.e. NOA_Sp-, absence of sperm in the testes). Thus, the second aspect of the invention also refers to an *in vitro* method for predicting the presence of sperm in an azoospermic subject, comprising the analysis of the expression profile of a predetermined set of miRNAs in a biological sample taken from said subject, wherein said predetermined set of miRNAs comprises, preferably consists of, miR-31-5p and miR-941. More preferably, the method further comprises the analysis of the FSH value in a blood sample taken from said subject.

In a preferred embodiment according to any one of the embodiments described in the first and second aspect of the invention, miR-539-5p, miR-941, miR-31-5p, miR-205-5p, miR-182-3p, miR-192-3p, miR-19a-3p, miR-200a-3p, miR-23b-5p, miR-363-3p, miR-451a, miR-934 are hsa-miR-539-5p (SEQ ID NO 1), hsa-miR-941 (SEQ ID NO 2), hsa-miR-31-5p (SEQ ID NO 3), hsa-miR-205-5p (SEQ ID NO 4), hsa-miR-182-3p (SEQ ID NO 5), hsa-miR-192-3p (SEQ ID NO 6), hsa-miR-19a-3p (SEQ ID NO 7), hsa-miR-200a-3p (SEQ ID NO 8), hsa-miR-23b-5p (SEQ ID NO 9), hsa-miR-363-3p (SEQ ID NO 10), hsa-miR-451a (SEQ ID NO 11) and hsa-miR-934 (SEQ ID NO 12), respectively.

In another preferred embodiment according to any one of the embodiments described in the first and second aspect of the invention, the expression profile is analysed using a multivariate regression analysis. More preferably, the analysis is carried out using a multivariate regression model, e.g. using formula I.

The findings of the inventors contribute to the search for the most valuable genetic markers which are potentially useful as tools for determining the origin of azoospermia and/or for predicting the presence of residual spermatogenesis. Thus, the miRNAs of the present invention are seminal markers for azoospermia representing a promising alternative or an additional test to traditional markers. Such information would be useful in the clinics, helping in the diagnosis of azoospermic patients but also providing realistic information about the chances of sperm retrieval from the testes in order to avoid unnecessary biopsies.

Thus, in a **third aspect**, the present invention refers to a set of miRNAs for use in the prediction of the presence of sperm in the testes of a subject with non-obstructive azoospermia, wherein the set of miRNAs comprises miR-539-5p and miR-941 and is isolated from a biological sample of said subject. In particular, it refers to a set of miRNAs as a marker of the prediction, preferably the *in vitro* prediction, of the presence of sperm in the testes of a subject with non-obstructive azoospermia, wherein the set of miRNAs comprises miR-539-5p and miR-941, and is isolated from a biological sample of said subject. More particularly, it refers to the use of a set of miRNAs for the prediction of the presence of sperm in the testes of a subject with non-obstructive azoospermia, wherein the set of miRNAs comprises miR-539-5p and miR-941 and is isolated from a biological sample of said subject. Preferably, the set of miRNAs consists of miR-539-5p and miR-941.

The third aspect of the invention also refers to a set of miRNAs for use in the discrimination between NOA and OA, wherein the set of miRNAs comprises one or more (e.g. all) miRNAs selected from the group consisting of miR-539-5p, miR-941, miR-31-5p, miR-205-5p, miR-182-3p, miR-192-3p, miR-19a-3p, miR-200a-3p, miR-23b-5p, miR-363-3p, miR-451a, miR-934 and combinations thereof and is isolated from a biological sample taken from an azoospermic subject. Preferably the set of miRNAs comprises miR-539-5p or miR-31-5p or miR-205-5p or combinations thereof, and more preferably the set of miRNA comprises or consists of miR-31-5p and/or miR-205-5p, and even more preferably the set comprises or consists of miR-31-5p.

The third aspect of the invention also refers to a set of miRNAs for use in predicting the presence of sperm in the testes of a subject with azoospermia, wherein the set of miRNAs comprising one or more miRNA(s) selected from the group consisting of miR-539-5p, miR-941, miR-31-5p, miR-205-5p, miR-182-3p, miR-192-3p, miR-19a-3p, miR-200a-3p, miR-23b-5p, miR-363-3p, miR-451a, miR-934 and combinations thereof, and wherein the set of miRNAs is isolated from a biological sample of said subject. In particular, it refers to said set of miRNAs as a marker of the prediction, preferably the *in vitro* prediction, of the presence of sperm in the testes of a subject with azoospermia. More particularly, it refers to the use of said set of miRNAs for the prediction of the presence of sperm in the testes of a subject with azoospermia.

In a preferred embodiment according to any one of the embodiments of the previous paragraph, the set of miRNAs comprises miR-31-5p, miR-205-5p, miR-941, miR-539-5p or combinations thereof. More preferably, it comprises miR-31-5p and miR-941, and optionally miR-539-5p. More preferably the set of miRNAs consists of miR-31-5p and miR-941, and optionally miR-539-5p.

In a preferred embodiment according to any one of the embodiments of the third aspect of the invention, miR-539-5p, miR-941, miR-31-5p, miR-205-5p, miR-182-3p, miR-192-3p, miR-19a-3p, miR-200a-3p, miR-23b-5p, miR-363-3p, miR-451a, miR-934 are hsa-miR-539-5p, hsa-miR-941, hsa-miR-31-5p, hsa-miR-205-5p, hsa-miR-182-3p, hsa-miR-192-3p, hsa-miR-19a-3p, hsa-miR-200a-3, hsa-miR-23b-5p, hsa-miR-363-3p, hsa-miR-451a and hsa-miR-934, respectively.

The method of any of the embodiments described in the first and second aspects of the present invention can be carried out using a kit. Thus, a first embodiment of a **fourth aspect** of the present invention refers to a kit to carry out the method according to any one of the embodiments of the first aspect of the invention, comprising:

a) appropriate means to determine the expression profile of a predetermined set of miRNA comprising or consisting of miR-539-5p and miR-941;
and a suitable packaging.

In a particular embodiment, the predetermined set of miRNAs further comprises or consists of one or more miRNAs selected from the group consisting of miR-31-5p, miR-205-5p, miR-182-3p, miR-192-3p, miR-19a-3p, miR-200a-3p, miR-23b-5p, miR-363-3p, miR-451a, miR-934 and combinations thereof, preferably miR-31-5p and/or miR-205-5p and more preferably miR-31-5p.

In a second embodiment of the fourth aspect of the invention, the invention refers to a kit to carry out the method according to any one of the embodiments of the second aspect of the invention, comprising:

a) appropriate means to determine the expression profile of a predetermined set of miRNA comprising one or more miRNAs selected from the group consisting of miR-539-5p, miR-941, miR-31-5p, miR-205-5p, miR-182-3p, miR-192-3p, miR-19a-3p, miR-200a-3p, miR-23b-5p, miR-363-3p, miR-451a, miR-934 and combinations thereof;
and a suitable packaging.

In a preferred embodiment according to the second embodiment of the fourth aspect of the invention, the predetermined set of miRNAs comprises or consists of miR-31-5p and/or miR-205-5p and/or miR-539-5p, and more preferably comprises or consists of miR-31-5p.

In a third embodiment of the fourth aspect of the invention, the invention refers to a kit for predicting the presence of sperm in the testes of an azoospermic subject, comprising:

a) appropriate means to determine the expression profile of a predetermined set of miRNA comprising miR-31-5p and miR-941, and optionally miR-539-5p;
and a suitable packaging.

In a preferred embodiment according to any one of the embodiments of the forth aspect of the invention, miR-539-5p, miR-941, miR-31-5p, miR-205-5p, miR-182-3p, miR-192-3p, miR-19a-3p, miR-200a-3p, miR-23b-5p, miR-363-3p, miR-451a, miR-934 are hsa-miR-539-5p, hsa-miR-941, hsa-miR-31-5p, hsa-miR-205-5p, hsa-miR-182-3p, hsa-miR-192-3p, hsa-miR-19a-3p, hsa-miR-200a-3, hsa-miR-23b-5p, hsa-miR-363-3p, hsa-miR-451a and hsa-miR-934, respectively.

In a particular embodiment of the kit according to any one of the preceding embodiments, the determination of the expression profile is carried out by any one of the methods indicated above in the "miRNA-(expression) profile" definition, thus the kit comprises the appropriate means for such determination.

In a particular embodiment of the kit according to any one of the preceding embodiments, the component a) comprises a set of polynucleotides for determining the expression profile of the predetermined set of miRNAs, wherein the polynucleotides are complementary to the nucleotide sequences of the miRNAs comprised in the predetermined set of miRNAs.

In a particular embodiment according to any one of the embodiments of the fourth aspect of the invention, the kit further comprises appropriate means to quantify the level of FSH in a blood sample or to compare the level of FSH in a blood sample with a reference or control FSH value.

In another particular embodiment according to any one of the embodiments of the fourth aspect of the invention, the kit further comprises a reference expression profile, more particularly it comprises positive and/or negative control samples.

Suitable kits may include primers for amplification and/or means for detection, and various reagents for use in accordance with the present invention in suitable containers and packaging materials, including tubes, vials, and shrink-wrapped and blow-moulded packages. Optionally, the kit includes sample preparation reagents and/or articles (e.g. tubes) to extract nucleic acids from samples. Optionally, the kits of the invention can contain instructions for the simultaneous, sequential or separate use of the different components which are in the kit. Optionally, the kits of the invention can contain appropriate means to extract RNA from a biological sample.

The kits according to the present invention make it possible to determine the origin of azoospermia and/or predict the presence of residual spermatogenesis in a subject with azoospermia, in particular a non-obstructive azoospermic subject, in an accurate and easy manner. Thus, a **fifth aspect** of the present invention refers to the use of a kit according to any one of the embodiments of the first embodiment of the fourth aspect of the invention, for predicting the presence of sperm in the testes of a subject with non-obstructive azoospermia.

The fifth aspect of the present invention also refers to the use of a kit according to any one of the embodiments of the second embodiment of the fourth aspect of the invention, for determining the origin azoospermia, i.e. for discriminating between NOA and OA in an azoospermic subject. Those subjects with OA have conserved spermatogenesis and therefore have sperm in the testes, being therefore the kit useful to predict the presence of sperm in the testes.

The fifth aspect of the present invention refers to the use of a kit according to any one of the embodiments of the third embodiment of the fourth aspect of the invention, for predicting the presence of sperm in the testes of a subject with azoospermia.

In a preferred embodiment according to any one of the embodiments of the fifth aspect of the invention, the use is an *in vitro* use.

While the foregoing invention has been described in some detail for purposes of clarity and understanding, it will be appreciated by one skilled in the art from a reading of this disclosure that various changes in form and detail can be made without departing from the true scope of the invention and appended claims.

The examples below serve to further illustrate the invention, and to provide those of ordinary skill in the art with a complete disclosure and description of how the methods and uses herein are carried out, and are not intended to limit the scope of the present invention.

EXAMPLES

**EXAMPLE 1.- Materials and Methods**

1.1.- Subjects of study

Patients and controls participating in the study were selected from men referred to the Andrology Service of the Fundació Puigvert. The study was approved by the Institutional Review Board of the Centre and all the participants signed a written informed consent. The methods were carried out in accordance with the approved guidelines.

Semen samples were obtained from 9 normozoospermic (Nz) fertile individuals consulting for vasectomy (control group); 14 infertile men diagnosed with non-obstructive azoospermia (NOA, no sperm in semen sample due to spermatogenic failure) or cryptozoospermia (Cr, $\leq 0.15 \times 10^6$ sperm/ml) (abbreviated as NOA in Tables 1-2, 4-5 and Figures 1-3); and 13 individuals with obstructive azoospermia (OA) and conserved spermatogenesis including both, men successfully vasectomized (OA-V, n=8) and individuals presenting pathological naturally occurring- obstruction in the genital tract (OA-N, n=5). Additionally, three severe oligozoospermic individuals (Oligo, $<5 \times 10^6$ sperm/ml) were included in the study (Table 1).

**Table 1.-** Clinical data of individuals included in the study.

| Patient No. | Spermio-gram | Sub-groups | Age[a] | Testes volume (R, L) | FSH (IU/L) | Semen Volume (mL) | pH (>7.2) | Sperm-count ($\times 10^6$/ mL) | Progres-sive motility (%) | Nomal morpholo-gy (%) | Fruc-tose (umol/ ejac) >13 | Citrate (umol/ ejac) >52 | a-glucosidase (mU/ ejac) >20 | Naturally con-ceived children (n) | IVF con-ceived children (n IVF cy-cles) | TESE val-ue (mil-lion/mL) |
|---|---|---|---|---|---|---|---|---|---|---|---|---|---|---|---|---|
| 1 | Nz | Nz | 41 | 20. 20 | nd | 2.5 | 7.5 | 137 | 47 | 15 | nd | nd | nd | Yes (2) | - | nd |
| 2 | Nz | Nz | 40 | 20. 20 | nd | 6.3 | 7.5 | 114 | 65 | 5 | nd | nd | nd | Yes (2) | - | nd |
| 3 | Nz | Nz | 40 | 20. 20 | nd | 2.4 | 7.5 | 113 | 52 | 6 | nd | nd | nd | Yes (2) | - | nd |
| 4 | Nz | Nz | 44 | 20. 20 | nd | 3.6 | 7.5 | 90 | 73 | 9 | nd | nd | nd | Yes (2) | - | nd |
| 5 | Nz | Nz | 39 | 15. 15 | nd | 4.9 | 7.2 | 60 | 53 | 6 | nd | nd | nd | Yes (1) | - | nd |
| 6 | Nz | Nz | 37 | 20. 20 | nd | 4.2 | 7.5 | 56 | 70 | 12 | nd | nd | nd | Yes (2) | - | nd |
| 7 | Nz | Nz | 46 | 15. 15 | nd | 2.8 | 7.7 | 41 | 42 | 4 | nd | nd | nd | Yes (2) | - | nd |
| 8 | Nz | Nz | 39 | 20. 20 | nd | 2.3 | 7.5 | 20 | 63 | 8 | nd | nd | nd | Yes (2) | - | nd |
| 9 | Nz | Nz | 45 | 22. 22 | nd | 4.1 | 7.2 | 20 | 36 | 9 | nd | nd | nd | Yes (2) | - | nd |
| 10 | AZO | NOA (Sp +) | 39 | 6. 9 | 12.6 | 6.5 | 7.5 | 0.15 | 13 | 0 | 47 | 84 | 15 | No | - | nd |
| 11 | AZO | NOA (Sp +) | 36 | 17. 0 | 15.3 | 4 | 7.2 | 0.1 | 4 | 0 | nd | nd | nd | No | No (1 IVF) | R: 0.029 |
| 12 | AZO | NOA (Sp +) | 48 | 20. 10 | 17.2 | 2.6 | 7.2 | 0.01 | 0 | 0 | nd | nd | nd | No | Gest (2 IVF) | R: 0.02; L: 001 |
| 13 | AZO | NOA* (Sp +) | 30 | 10.9 | 044 | 3.8 | 7.7 | 0.001 | 0 | 0 | 51 | 48 | 18 | No | - | nd |
| 14 | AZO | NOA* (Sp +) | 22 | 4. 4 | 0.5 | 1.7 | 7.7 | 0.001 | 0 | 0 | 50 | 56 | 18 | No | - | nd |
| 15 | AZO | NOA (Sp +) | 51 | 8. 8 | 19.29 | 3.3 | 7.2 | 0-0.001 | 0 | 0 | nd | nd | nd | No | - | nd |
| 16 | AZO | NOA (Sp +) | 30 | 10, 8 | 113 | 6 | 7.5 | 0 | 0 | 0 | 146 | 62 | 12 | No | Gest (1 IVF) | R+L: 0.001: μTESE L: 0.047 |

| Patient No. | Spermio-gram | Sub-groups | Age[a] | Testes volume (R, L) | FSH (IU/L) | Semen Volume (mL) | pH (>7.2 ) | Sperm-count ($\times 10^6$/ mL) | Progres-sive motility (%) | Nomal morpholo-gy (%) | Fruc-tose (umol/ejac) >13 | Citrate (umol/ejac) >52 | a-glucosidase (mU/ejac) >20 | Naturally con-ceived children (n) | IVF con-ceived children (n IVF cy-cles) | TESE val-ue (mil-lion/mL) |
|---|---|---|---|---|---|---|---|---|---|---|---|---|---|---|---|---|
| 17 | AZO | NOA (Sp +) | 45 | 15. 15 | 15 | 3.1 | 7.7 | 0 | - | - | 53 | 39 | 6 | No | No (1 IVF) | R: 007: L: 0.06 |
| 18 | AZO | NOA (Sp -) | 38 | 10, 10 | 8.45 | 4.7 | 7.5 | 0 | - | - | 35 | 73 | 10 | No | - | R:0; L: 0 |
| 19 | AZO | NOA (Sp -) | 45 | 10, 12 | 55 | 5.2 | 7.7 | 0 | - | - | nd | nd | nd | No | - | R: 0: L: 0002 |
| 20 | AZO | NOA (Sp -) | 30 | 9, 9 | 32.94 | 8.5 | 7.2 | 0 | - | - | 176 | 167 | 25 | No | - | R:0; L: 0 |
| 21 | AZO | NOA (Sp -) | 30 | 13, 15 | 18.4 | 5.1 | 7.5 | 0 | - | - | 156 | 83 | 13 | No | - | R:0; L: 0 |
| 22 | AZO | NOA | 34 | 12, 10 | 38 | 5.3 | 7.2 | 0 | - | - | nd | nd | nd | No | - | nd |
| 23 | AZO | NOA | 32 | 8. 8 | 18.23 | 10.8 | 7.2 | 0 | - | - | 161 | 321 | 23 | No | - | nd |
| 24 | AZO | OA-N [#] | 35 | 20, 20 | 10 | 1.3 | 6.4 | 0 | - | - | nd | nd | nd | No | - | R: 0.3: L: 0.55 |
| 25 | AZO | OA-N [#] | 37 | 25, 25 | nd | 0.5 | 6.4 | 0 | - | - | nd | nd | nd | No | - | R: 0.42; L: 0.25 |
| 26 | AZO | OA-N | 44 | 0, 15 | 8.38 | 2.9 | 7.5 | 0 | - | - | 48 | 93 | 31 | No | No (2 IVF) | L: 0.5 |
| 27 | AZO | OA-N | 33 | 0.20 | 6.6 | 4.5 | 7.2 | 0 | - | - | 57 | 97 | 3 | No | - | L: 0.182 |
| 28 | AZO | OA-N | 41 | 20, 20 | 1.73 | 2.1 | 7.5 | 0 | - | - | 86 | 69 | 12 | No | Gest (2 IVF) | R: 0.246 |
| 29 | AZO | OA-V | 41 | 15. 15 | - | 1.8 | 7.5 | 0 | - | - | nd | nd | nd | Yes (2) | - | nd |
| 30 | AZO | OA-V | 40 | 15. 15 | - | 2.7 | 7.7 | 0 | - | - | nd | nd | nd | Yes (2) | - | nd |
| 31 | AZO | OA-V | 40 | 25. 25 | - | 3 | 7.7 | 0 | - | - | nd | nd | nd | Yes (1) | - | nd |
| 32 | AZO | OA-V | 40 | 20. 20 | - | 2.7 | 7.5 | 0 | - | - | nd | nd | nd | Yes (2) | - | nd |

| Patient No. | Spermio-gram | Sub-groups | Age[a] | Testes volume (R, L) | FSH (IU/L) | Semen Volume (mL) | pH (>7.2 ) | Sperm-count (×$10^6$/ mL) | Progres-sive motility (%) | Nomal morpholo-gy (%) | Fruc-tose (umol/ ejac) >13 | Citrate (umol/ ejac) >52 | a-glucosidase (mU/ ejac) >20 | Naturally con-ceived children (n) | IVF con-ceived children (n IVF cy-cles) | TESE val-ue (mil-lion/mL) |
|---|---|---|---|---|---|---|---|---|---|---|---|---|---|---|---|---|
| 33 | AZO | OA-V | 40 | 20. 20 | - | 2.7 | 7.5 | 0 | - | - | nd | nd | nd | Yes (2) | - | nd |
| 34 | AZO | OA-V | 39 | 20. 20 | - | 4.2 | 7.2 | 0 | - | - | nd | nd | nd | Yes (2) | - | nd |
| 35 | AZO | OA-V | 36 | 25. 25 | - | 17 | 7.7 | 0 | - | - | nd | nd | nd | Yes (2) | - | nd |
| 36 | AZO | OA -V | 40 | 25. 25 | - | 1.9 | 7.5 | 0 | | - | nd | nd | nd | Yes (2) | - | nd |
| 37 | OLIGO | Oligo | 37 | 20, 20 | 4.82 | 3.8 | 7.7 | 2 | 54 | 10 | nd | nd | nd | No | No (1 IVF) | nd |
| 38 | OLIGO | Oligo | 35 | 15. 9 | 3.8-4.2 | 4.1 | 7.2 | 3 | 40 | 4 | nd | nd | nd | No | - | nd |
| 39 | OLIGO | Oligo | 21 | 15, 12 | 2.53 | 6 | 7.5 | 5 | 9 | 0 | nd | nd | nd | No (single) | - | nd |

R: right testis; L: left testis.

[a] :age at the time of clinical assessment.

* hypogonadism.

# CBAVD (congenital bilateral absence of the vas deferens).

EP 3 543 358 A1

Semen analysis was performed on all the individuals and, for their interpretation, reference normal value ranges for semen analyses were in accordance with World Health Organization guidelines (Cooper et al., World Health Organization reference values for human semen characteristics. Hum Reprod Update 2010; 16: 231-245).

The routine clinical and genetic procedures for infertile patients included medical history, physical examination, hormonal study, histological evaluation of a testicular biopsy, karyotype and analysis of Y-chromosome microdeletions. Men with spermatogenic disorders included in the study did not show clinical factors (varicocele, infection, immunologic factors, anatomic malformation, or chemical insults) or genetic causes (chromosomal aberration or a Y-chromosome microdeletion) for their infertility.

For the subsequent analysis of exosome miRNA levels in semen, samples were used into a two-stages (screening and validating stages) case-control designed study (Figure 1).

1.2.- Semen samples and exosome isolation

Semen samples were obtained by masturbation after 3-5 days of sexual abstinence. They were allowed to liquefy for 30 min at 37°C. Isolation of exosomal vesicles was performed by differential centrifugation steps including one microfiltration step and ultracentrifugation as described elsewhere (Crescitelli et al., 2013; Li *et al.*, 2012). Briefly, cells and apoptotic bodies were pelleted by centrifugation at 1600 x g for 10 minutes and microvesicles at 16,500 x g for 10 minutes at 4°C to obtain seminal plasma (supernatant) which was carefully collected and immediately stored at -80°C until use.

Seminal plasma (200 $\mu$l) was filtered (0.22 $\mu$m pore size) to remove macromolecules and cell debris that they may have still contained. Subsequently, 9 ml of PBS was additionally filtrated. The resulting filtrate was ultracentrifuged at 100000 xg in a SW40 rotor for 2h at 4°C to sediment the sEVs, which mainly contain exosomes. The pellet was resuspended in 100 $\mu$l PBS and frozen at -80°C. Nanoparticle tracking analysis was performed by NANOSIGHT showing an enrichment of particles with a size of <200nm, accounting for 95% of the particles recovered. The vesicle distribution by size and the morphological parameters presented no significant differences among groups.

1.3.- Small RNA-containing total RNA isolation

The exosome suspension was treated with RNAse A (Qiagen) (100 $\mu$g/ml final reaction concentration) by incubating the sample for 15 min at 37°C to degrade the residual RNA outside the vesicles.

Total RNA was obtained from exosomes using the miRCURY RNA Isolation Kit-Cell and Plant (Exiqon), according to the instructions specifically provided by the manufacturer for RNA preparation from exosome suspension. RNA was eluted in 50 $\mu$l of elution buffer. RNA concentration was calculated by using the QUBIT fluorometer and the Quant-iT RNA Assay kit (Invitrogen). All RNA samples from seminal plasma exosomes presented an OD 260/280 nm ratio $\geq$1.7 when using a Nanodrop microvolume UV-Vis spectrophotometer (Thermo Scientific).

1.4.- Exosomal miRNA qPCR profiling

For the miRNA screening, 19 $\mu$l RNA was reverse transcribed in 95 $\mu$l reactions using the miRCURY LNA™ Universal RT microRNA PCR, Polyadenylation and cDNA synthesis kit (Exiqon). cDNA was diluted 50x and assayed in 10 $\mu$l PCR reactions according to the protocol for miRCURY LNA™ Universal RT microRNA PCR; each miRNA was assayed once by qPCR on the microRNA Ready-to-Use PCR Human panels I and II that include 623 mature miRNAs of miRBase. Negative controls excluding the template from the reverse transcription reaction were performed and profiled like the samples. cDNA and ExiLENT SYBR Green mastermix were transferred to qPCR panels preloaded with primers, using a pipetting robot. The amplification was performed in a LightCycler® 480 Real-Time PCR System (Roche) in 384 well plates. The amplification curves were analyzed using the Roche LC software, both for determination of Crossing Points-Cp (by the 2nd derivative method) and for melting curve analysis. The amplification efficiency was calculated using algorithms similar to the LinReg software. All assays were inspected for distinct melting curves and the Tm was checked to be within known specifications for the assay. Experiments were conducted at Exiqon Services.

Next, to correct for potential overall differences between the samples, for each sample, raw data (Cp values) were normalized to the mean of the 50 most stable assays (mean 50) that were detected in all samples: dCp = mean 50 Cp - assay Cp. Those assays were previously selected as being the ones that presented the lowest coefficient of variation (CV < 0.018) of Cp values among samples in the study (Table 2) as well as no statistical differences in absolute expression levels between groups, either individually or the mean value.

The relative quantitative method of $2^{dCp}$ was used to calculate the relative quantification (RQ) miRNA expression values.

**Table 2.-** List of the 70 miRNAs showing the most stable expression among samples. The 50 most stable miRNAs (in bold) were selected to normalize data from miRNA profiling. hsa-miR-30e-5p and hsa-miR-30d-5p (in italic) were selected to normalize data from candidate miRNAs.

**Table 2.-** List of the 70 miRNAs showing the most stable expression among samples

| miRNAs | Nz1 Cp value | Nz2 Cp value | Nz3 Cp value | OA1 Cp value | OA2 Cp value | OA3 Cp value | NOA1 Cp value | NOA2 Cp value | NOA3 Cp value | mean (Cp value) | SD (Cp value) | CV (Cp value) |
|---|---|---|---|---|---|---|---|---|---|---|---|---|
| average 50 stables miRNAs (selected to normalize) | 29.12 | 29.22 | 29.20 | 28.54 | 29.22 | 29.47 | 28.99 | 29.37 | 28.67 | 29.09 | 0.31 | 0.0106 |
| hsa-miR-200a-5p | 29.59 | 29.71 | 29.91 | 29.65 | 30.13 | 30.08 | 29.67 | 29.70 | 29.46 | 29.77 | 0.22 | 0.0075 |
| hsa-let-7b-3p | 29.57 | 29.51 | 29.45 | 29.31 | 29.53 | 29.72 | 29.66 | 29.90 | 29.16 | 29.53 | 0.22 | 0.0074 |
| hsa-miR-141-5p | 28.97 | 29.18 | 28.99 | 28.47 | 29.03 | 29.10 | 28.80 | 29.09 | 28.56 | 28.91 | 0.25 | 0.0086 |
| hsa-miR-664a-3p | 30.10 | 29.70 | 29.95 | 29.77 | 30.02 | 29.95 | 29.80 | 30.50 | 29.72 | 29.95 | 0.25 | 0.0083 |
| hsa-miR-20a-3p | 29.08 | 29.30 | 28.98 | 28.55 | 28.83 | 29.16 | 28.68 | 29.25 | 28.57 | 28.93 | 0.29 | 0.0099 |
| hsa-miR-200c-5p | 31.58 | 31.74 | 31.89 | 31.52 | 32.43 | 31.71 | 31.90 | 31.46 | 31.12 | 31.71 | 0.36 | 0.0114 |
| hsa-miR-339-5p | 28.34 | 28.12 | 28.03 | 27.47 | 28.00 | 28.23 | 27.84 | 28.46 | 27.51 | 28.00 | 0.34 | 0.0123 |
| hsa-miR-590-3p | 31.23 | 31.70 | 31.05 | 30.89 | 30.80 | 31.50 | 30.78 | 31.17 | 30.68 | 31.09 | 0.35 | 0.0111 |
| hsa-miR-425-5p | 27.55 | 27.82 | 27.81 | 27.09 | 27.82 | 27.90 | 27.35 | 27.82 | 27.28 | 27.60 | 0.30 | 0.0108 |
| hsa-miR-93-3p | 29.77 | 29.27 | 30.04 | 28.79 | 29.79 | 29.81 | 29.51 | 29.47 | 29.15 | 29.51 | 0.39 | 0.0132 |
| hsa-miR-671-3p | 31.79 | 32.16 | 32.22 | 31.64 | 32.36 | 32.87 | 31.95 | 32.57 | 31.66 | 32.13 | 0.42 | 0.0131 |
| hsa-miR-1468-5p | 32.35 | 32.32 | 33.07 | 31.99 | 32.54 | 32.76 | 33.00 | 32.76 | 31.85 | 32.51 | 0.42 | 0.0130 |
| hsa-miR-99b-3p | 31.70 | 31.29 | 31.63 | 30.85 | 32.12 | 32.07 | 31.44 | 31.92 | 31.33 | 31.60 | 0.41 | 0.0130 |
| hsa-miR-505-3p | 29.89 | 30.30 | 30.27 | 29.64 | 30.62 | 30.55 | 29.76 | 30.63 | 29.67 | 30.15 | 0.41 | 0.0137 |
| hsa-let-7e-3p | 32.46 | 32.79 | 32.43 | 31.55 | 32.68 | 33.16 | 32.16 | 32.73 | 32.75 | 32.52 | 0.46 | 0.0141 |
| hsa-miR-26b-3p | 32.30 | 32.16 | 31.56 | 31.27 | 31.63 | 31.82 | 31.31 | 32.09 | 31.12 | 31.69 | 0.42 | 00134 |
| hsa-miR-425-3p | 29.84 | 29.92 | 29.75 | 29.03 | 30.04 | 30.06 | 29.56 | 30.04 | 29.38 | 29.73 | 0.35 | 0.0118 |
| hsa-miR-590-5p | 27.52 | 27.53 | 27.72 | 27.00 | 27.79 | 28.20 | 27.57 | 27.84 | 26.99 | 27.57 | 0.39 | 0.0141 |
| hsa-miR-362-3p | 29.87 | 30.31 | 29.67 | 29.48 | 30.05 | 30.53 | 30.03 | 30.50 | 29.74 | 30.02 | 0.37 | 0.0124 |
| hsa-miR-362-3p | 29.87 | 30.31 | 29.67 | 29.48 | 30.05 | 30.53 | 30.03 | 30.50 | 29.74 | 30.02 | 0.37 | 0.0124 |
| hsa-miR-7-1-3p | 28.99 | 28.65 | 28.52 | 28.48 | 29.07 | 29.15 | 28.24 | 29.24 | 28.64 | 28.78 | 0.35 | 0.0121 |
| hsa-miR-24-1-5p | 33.59 | 34.29 | 33.17 | 32.89 | 32.95 | 33.70 | 32.97 | 33.36 | 32.80 | 33.30 | 0.49 | 0.0146 |
| hsa-miR-491-5p | 29.71 | 29.91 | 29.83 | 29.11 | 29.78 | 30.22 | 29.58 | 29.94 | 28.80 | 29.65 | 0.44 | 0.0149 |
| hsa-miR-339-3p | 29.45 | 29.08 | 29.48 | 28.49 | 29.46 | 29.64 | 29.24 | 29.76 | 28.74 | 29.26 | 0.42 | 0.0144 |
| hsa-miR-219a-5p | 31.77 | 32.06 | 32.12 | 31.20 | 31.03 | 32.25 | 31.60 | 32.02 | 31.54 | 31.73 | 0.42 | 0.0133 |
| hsa-miR-19b-3p | 22.82 | 22.72 | 22.64 | 22.06 | 22.63 | 22.90 | 22.62 | 23.09 | 22.12 | 22.62 | 0.34 | 0.0149 |
| hsa-miR-30c-5p | 23.86 | 24.04 | 23.90 | 23.28 | 23.76 | 24.45 | 23.85 | 24.26 | 23.54 | 23.88 | 0.35 | 0.0147 |
| hsa-miR-744-3p | 33.07 | 32.92 | 32.50 | 32.22 | 33.14 | 33.58 | 32.53 | 32.88 | 32.00 | 32.76 | 0.49 | 0.0150 |

| miRNAs | Nz1 Cp value | Nz2 Cp value | Nz3 Cp value | OA1 Cp value | OA2 Cp value | OA3 Cp value | NOA1 Cp value | NOA2 Cp value | NOA3 Cp value | mean (Cp value) | SD (Cp value) | CV (Cp value) |
|---|---|---|---|---|---|---|---|---|---|---|---|---|
| hsa-miR-1296-5p | 31.31 | 31.37 | 31.05 | 30.34 | 31.45 | 31.48 | 30.97 | 30.94 | 30.37 | 31.03 | 0.43 | 0.0139 |
| hsa-miR-429 | 26.23 | 26.93 | 26.68 | 25.91 | 26.31 | 26.79 | 26.03 | 26.91 | 26.08 | 26.43 | 0.40 | 0.0152 |
| hsa-miR-374b-5p | 26.28 | 26.43 | 26.61 | 25.73 | 26.45 | 26.76 | 26.51 | 26.69 | 25.70 | 26.35 | 0.39 | 0.0147 |
| hsa-let-7f-2-3p | 31.77 | 31.98 | 31.25 | 31.08 | 31.09 | 31.11 | 31.28 | 32.12 | 31.18 | 31.43 | 0.41 | 0.0131 |
| hsa-miR-582-5p | 27.15 | 27.47 | 28.25 | 27.54 | 27.90 | 27.67 | 27.48 | 28.05 | 27.28 | 27.64 | 0.36 | 0.0130 |
| hsa-miR-345-5p | 27.51 | 27.85 | 27.63 | 27.14 | 28.10 | 28.13 | 27.43 | 27.95 | 27.06 | 27.64 | 0.39 | 0.0142 |
| hsa-miR-140-5p | 29.45 | 30.04 | 29.75 | 28.86 | 29.55 | 30.50 | 29.61 | 29.45 | 29.30 | 29.61 | 0.46 | 0.0156 |
| hsa-miR-200a-3p | 23.48 | 23.96 | 23.84 | 23.17 | 23.99 | 24.26 | 23.47 | 23.76 | 23.24 | 23.69 | 0.37 | 0.0155 |
| hsa-miR-374a-5p | 27.50 | 28.25 | 28.05 | 26.93 | 27.55 | 27.95 | 27.84 | 28.12 | 27.14 | 27.70 | 0.45 | 0.0163 |
| hsa-miR-101-3p | 24.22 | 24.69 | 24.33 | 23.87 | 24.48 | 24.99 | 24.00 | 24.81 | 24.00 | 24.38 | 0.39 | 0.0162 |
| hsa-miR-200b-3p | 22.12 | 21.96 | 22.19 | 21.60 | 22.45 | 22.63 | 21.94 | 22.00 | 21.59 | 22.05 | 0.35 | 0.0157 |
| hsa-miR-19a-3p | 25.07 | 25.63 | 25.32 | 24.66 | 25.13 | 25.66 | 25.47 | 25.93 | 24.91 | 25.31 | 0.40 | 0.0160 |
| hsa-miR-30b-5p | 23.24 | 23.37 | 23.29 | 22.82 | 23.24 | 23.86 | 23.30 | 23.80 | 22.82 | 23.30 | 0.36 | 0.0155 |
| hsa-miR-33b-3p | 31.90 | 31.66 | 32.26 | 31.47 | 32.01 | 32.15 | 33.04 | 32.24 | 32.04 | 32.09 | 0.45 | 0.0139 |
| hsa-miR-532-3p | 27.76 | 27.79 | 28.15 | 27.36 | 28.10 | 28.70 | 27.88 | 28.12 | 27.52 | 27.93 | 0.40 | 0.0142 |
| hsa-miR-629-3p | 34.23 | 33.10 | 33.86 | 32.80 | 33.24 | 33.18 | 32.97 | 33.96 | 33.19 | 33.39 | 0.50 | 0.0149 |
| hsa-miR-598-3p | 29.19 | 28.82 | 29.07 | 28.58 | 29.54 | 29.53 | 28.99 | 29.10 | 27.97 | 28.98 | 0.49 | 0.0168 |
| hsa-miR-29b-2-5p | 28.74 | 29.04 | 28.91 | 28.04 | 29.09 | 29.47 | 28.58 | 29.13 | 28.18 | 28.80 | 0.47 | 0.0162 |
| hsa-miR-128-3p | 30.28 | 31.47 | 30.86 | 29.84 | 30.37 | 30.71 | 30.00 | 30.58 | 30.14 | 30.47 | 0.50 | 0.0164 |
| hsa-miR-29a-5p | 28.68 | 28.12 | 28.25 | 27.71 | 28.24 | 28.83 | 28.10 | 29.05 | 27.80 | 28.31 | 0.46 | 0.0161 |
| hsa-miR-24-2-5p | 32.61 | 32.65 | 32.94 | 31.45 | 33.52 | 33.03 | 32.48 | 32.75 | 32.93 | 32.71 | 0.56 | 0.0172 |
| hsa-miR-20b-3p | 30.01 | 29.95 | 30.29 | 28.92 | 30.24 | 29.90 | 30.34 | 30.28 | 29.24 | 29.91 | 0.50 | 0.0168 |
| hsa-miR-301a-3p | 30.32 | 29.99 | 30.61 | 29.65 | 30.93 | 31.29 | 30.60 | 30.36 | 29.71 | 30.39 | 0.54 | 0.0179 |
| hsa-miR-335-5p | 29.78 | 30.37 | 30.27 | 29.77 | 30.37 | 31.19 | 29.65 | 30.30 | 29.62 | 30.15 | 0.50 | 0.0167 |
| hsa-miR-126-5p | 32.66 | 33.73 | 33.80 | 32.90 | 32.95 | 33.69 | 33.72 | 33.77 | 33.07 | 33.36 | 0.46 | 0.0137 |
| hsa-miR-141-3p | 21.62 | 21.67 | 21.62 | 20.96 | 21.63 | 22.06 | 21.28 | 21.94 | 21.18 | 21.55 | 0.35 | 0.0164 |
| hsa-miR-186-5p | 29.81 | 30.47 | 30.45 | 29.52 | 29.69 | 30.62 | 29.99 | 30.76 | 29.79 | 30.12 | 0.46 | 0.0151 |
| *hsa-miR-30e-5p* | 24.78 | 24.49 | 24.68 | 23.93 | 24.78 | 25.44 | 24.66 | 24.89 | 24.21 | 24.65 | 0.43 | 0.0173 |
| hsa-miR-200b-5p | 27.47 | 26.70 | 27.62 | 26.80 | 27.74 | 27.92 | 27.33 | 27.11 | 26.78 | 27.27 | 0.45 | 0.0165 |
| hsa-let-7d-3p | 27.87 | 29.27 | 28.60 | 27.82 | 28.53 | 28.74 | 27.91 | 28.43 | 27.99 | 28.35 | 0.49 | 0.0174 |
| hsa-miR-25-3p | 26.10 | 25.45 | 25.95 | 25.34 | 26.13 | 26.56 | 25.96 | 25.73 | 25.07 | 25.81 | 0.46 | 0.0179 |
| hsa-miR-26a-1-3p | 34.55 | 33.50 | 33.85 | 33.35 | 34.02 | 34.15 | 33.31 | 34.50 | 32.80 | 33.78 | 0.59 | 0.0174 |

(continued)

| miRNAs | Nz1 *Cp* value | Nz2 *Cp* value | Nz3 *Cp* value | OA1 *Cp* value | OA2 *Cp* value | OA3 *Cp* value | NOA1 *Cp* value | NOA2 *Cp* value | NOA3 *Cp* value | mean (*Cp* value) | SD (*Cp* value) | CV (*Cp* value) |
|---|---|---|---|---|---|---|---|---|---|---|---|---|
| hsa-miR-193a-5p | 29.43 | 29.03 | 29.54 | 28.66 | 29.13 | 29.79 | 28.82 | 29.64 | 28.38 | 29.16 | 0.48 | 0.0164 |
| hsa-miR-18b-5p | 28.27 | 28.80 | 29.05 | 27.52 | 28.73 | 28.70 | 28.52 | 28.59 | 27.80 | 28.44 | 0.49 | 0.0174 |
| hsa-miR-99b-5p | 25.66 | 25.59 | 26.18 | 24.99 | 26.22 | 26.27 | 25.49 | 26.33 | 25.47 | 25.80 | 0.47 | 0.0181 |
| hsa-miR-125b-2-3p | 29.12 | 29.07 | 29.38 | 28.50 | 29.89 | 29.77 | 28.96 | 29.80 | 28.66 | 29.24 | 0.50 | 0.0172 |
| hsa-miR-16-2-3p | 33.73 | 34.29 | 33.27 | 33.01 | 33.47 | 33.81 | 33.57 | 34.53 | 32.65 | 33.59 | 0.59 | 0.0176 |
| hsa-miR-454-3p | 29.48 | 29.61 | 29.66 | 28.83 | 29.85 | 30.28 | 29.73 | 29.97 | 28.66 | 29.56 | 0.52 | 0.0176 |
| hsa-miR-324-3p | 27.52 | 27.78 | 28.00 | 26.64 | 27.49 | 27.94 | 27.50 | 27.86 | 26.82 | 27.50 | 0.48 | 0.0175 |
| hsa-miR-18a-5p | 28.69 | 29.15 | 29.77 | 28.04 | 29.01 | 29.30 | 28.73 | 28.95 | 28.56 | 28.91 | 0.49 | 0.0169 |
| hsa-miR-148b-5p | 32.18 | 32.57 | 32.88 | 31.96 | 33.21 | 33.36 | 32.54 | 32.22 | 31.98 | 32.54 | 0.51 | 0.0158 |
| hsa-miR-19b-1-5p | 32.02 | 32.68 | 32.35 | 31.54 | 32.69 | 32.67 | 32.54 | 32.11 | 31.30 | 32.21 | 0.51 | 0.0160 |
| *hsa-miR-30d-5p* | 23.91 | 23.84 | 24.31 | 23.24 | 24.00 | 24.65 | 23.93 | 24.24 | 23.45 | 23.95 | 0.43 | 0.0179 |

## 1.5.- Validation of miRNA candidates by RT-qPCR analysis

First-stranded cDNA specific for miRNA was obtained by reverse transcription (RT) of 50 ng of RNA in 10 $\mu$l, using the Universal cDNA synthesis kit II (Exiqon). The resulting cDNA solution was stored at - 20°C. For quantitative real-time PCR (qPCR) analysis cDNAwas diluted (12x) and assayed in 10 $\mu$l PCR reactions containing ExiLENT SYBR Green master mix according to the protocol for miRCURY LNA™ Universal RT microRNA PCR (Exiqon). Duplicate amplification reactions of individual assays (LNA™-enhanced miRNA qPCR primers) were carried out on a LC96 (Lightcycler® 96 Real-time PCR Instrument; Roche) with the primers shown in Table 3. The qPCR cycle conditions were as follows:

- Polymerase Activation/Denaturation: 95°C, 10 min

- 40 amplification cycles, being each cyle: 95°C, 10 s; 60°C, 1 min (Ramp-rate 1.6°C/s).

**Table 3.-** List of the miRNA qPCR primers set.

| miRNA (SEQ ID NO) | Product number of primers set [a] |
|---|---|
| hsa-miR-182-3p (SEQ ID NO 5) | 204098 |
| hsa-miR-192-3p (SEQ ID NO 6) | 204272 |
| hsa-miR-205-5p (SEQ ID NO 4) | 204487 |
| hsa-miR-30d-5p (SEQ ID NO 13) | 206047 |
| hsa-miR-30e-3p (SEQ ID NO 14) | 204410 |
| hsa-miR-31-5p (SEQ ID NO 3) | 204236 |
| hsa-miR-539-5p (SEQ ID NO 1) | 205656 |
| hsa-miR-934 (SEQ ID NO 12) | 206086 |
| hsa-miR-941 (SEQ ID NO 2) | 204574 |
| [a] *miRCURY LNA™ Universal RT microRNA PCR LNA™ PCR primers set* (Exiqon) | |

Target miRNA expression for exosomes in semen samples was calculated relative to the expression of miR-30e-3p and miR-30d-5p, chosen from the exosomal miRNA qPCR profiling study among the most stable miRNAs (Table 2, see above). For normalization of data we applied the mean of the of miR-30e-3p and miR-30d-5p assays that were detected in all samples. The RQ values were calculated using the $2^{dCp}$ strategy. The same procedure was applied to determine tissular expression profiling of miRNA candidates.

## 1.6.- Statistical analysis

The non-parametric Kruskal-Wallis test was used to analyze the differences in clinical data and absolute expression levels of reference genes. Unpaired two-tailed t test was used to analyze the differences in relative expression of miRNAs in each patient group compared with controls in the miRNA profiling study. The non-parametric Mann-Whitney U-test was used to evaluate differences in relative expression of selected miRNAs in each patient group compared with controls. Pearson product-moment correlation coefficients (PCC) were calculated to determine the correlation between the miRNAs RQ values and the various parameters in semen analysis or testicular biopsy in patient groups and controls. Multivariate binary logistic regression (backward stepwise, conditional, method) and receiver operating characteristic (ROC) curve analysis of the relative expression values was used to distinguish the etiology of azoospermia, as well as individuals with positive TESE value > $0.01 \times 10^6$ sperm/ml (NOA_Sp+). Accuracy was measured as the area under the ROC curve (AUC). A $p$-value <0.05 was considered significant.

**EXAMPLE 2.- Obstructive and secretory azoospermia show altered profiles of miRNA contained in SP exosomes**

In order to identify global changes of the exosome miRNA levels in seminal plasma associated with azoospermia of different origin, the level of expression of 623 human miRNAs in SP exosomes from 3 normozoospermic individuals (Nz; sample no. 1,3,4) who showed normal characteristics in a seminogram, and from azoospermic men resulting from vasectomy surgical procedure (OA-V; n=3; sample no. 29-31) or resulting from impaired sperm production (NOA; n=3; sample no. 11, 16, 17) were first analyzed.

No amplification values were obtained for 78 miRNAs suggesting that the miRNA levels were beneath the detection threshold of the technique. Of the amplified miRNAs, 148 were excluded for further analysis due to poor amplification efficiency across samples (missing expression values for 6 out of the 9 samples). The remaining miRNAs (n=397; 63.7%) were further statistically evaluated and the results are presented in Table 4.

**Table 4.-** Summary of miRNA expression data in OA and in NOA phenotypes related to Nz. Statistically increased miRNA expression level depicted in light grey and statistically decreased miRNA expression level depicted in dark grey and italic, when compared with

controls. Cp values >38 are depicted in bold. * p<0.05; ** p<0.005. Statistically significant p-values are depicted in bold.

| miRNA | Average $Cp_{Nz}$ | Average $Cp_{OA}$ | Average $Cp_{NOA}$ | miRNA expression | | | p-value | |
|---|---|---|---|---|---|---|---|---|
| | | | | Nz | OA | NOA | OA-Nz | NOA-Nz |
| hsa-let-7a-5p | 20.57 | 20.30 | 20.17 | 1 | 1.124 | 1.173 | > 0.10 | > 0.10 |
| hsa-let-7b-3p | 29.51 | 29.52 | 29.57 | 1 | 0.926 | 0.853 | > 0.10 | 0.0518 |
| hsa-let-7b-5p | 21.54 | 21.35 | 21.25 | 1 | 1.063 | 1.088 | > 0.10 | > 0.10 |
| hsa-let-7c-5p | 23.01 | 22.63 | 22.60 | 1 | 1.215 | 1.185 | > 0.10 | > 0.10 |
| hsa-let-7d-3p | 28.58 | 28.36 | 28.11 | 1 | 1.085 | 1.233 | > 0.10 | > 0.10 |
| hsa-let-7d-5p | 27.16 | 27.13 | 26.79 | 1 | 0.957 | 1.149 | > 0.10 | > 0.10 |
| hsa-let-7e-3p | 32.56 | 32.46 | 32.55 | 1 | 0.997 | 0.897 | > 0.10 | > 0.10 |
| hsa-let-7e-5p | 25.56 | 25.26 | 25.10 | 1 | 1.146 | 1.223 | > 0.10 | > 0.10 |
| hsa-let-7f-1-3p | 34.25 | 34.02 | 33.23 | 1 | 1.093 | 1.808* | > 0.10 | **0.0370** |
| hsa-let-7f-2-3p | 31.66 | 31.09 | 31.53 | 1 | 1.388 | 0.978 | > 0.10 | > 0.10 |
| hsa-let-7f-5p | 24.47 | 24.02 | 23.70 | 1 | 1.269 | 1.514 | > 0.10 | > 0.10 |
| hsa-let-7g-5p | 23.48 | 23.28 | 22.92 | 1 | 1.068 | 1.311 | > 0.10 | > 0.10 |
| hsa-let-7i-3p | 36.30 | 35.97 | 35.56 | 1 | 1.183 | 1.514 | > 0.10 | > 0.10 |
| hsa-let-7i-5p | 27.92 | 27.70 | 27.40 | 1 | 1.087 | 1.277 | > 0.10 | > 0.10 |
| hsa-miR-100-5p | 27.78 | 28.24 | 28.45 | 1 | 0.675 | 0.557 | > 0.10 | > 0.10 |
| hsa-miR-101-3p | 24.41 | 24.45 | 24.27 | 1 | 0.911 | 0.980 | > 0.10 | > 0.10 |
| hsa-miR-101-5p | 32.12 | 31.73 | 31.66 | 1 | 1.221 | 1.222 | > 0.10 | > 0.10 |
| hsa-miR-103a-3p | 23.06 | 22.85 | 22.70 | 1 | 1.079 | 1.136 | > 0.10 | > 0.10 |
| hsa-miR-106a-3p[5cX] | 33.74 | 33.10 | 32.83 | 1 | 1.451 | 1.674 | > 0.10 | > 0.10 |
| hsa-miR-106a-5p[5cX] | 22.54 | 22.48 | 22.45 | 1 | 0.972 | 0.952 | > 0.10 | > 0.10 |
| hsa-miR-106b-3p | 31.44 | 31.09 | 30.86 | 1 | 1.190 | 1.334 | > 0.10 | > 0.10 |
| hsa-miR-106b-5p | 26.35 | 25.86 | 25.88 | 1 | 1.311 | 1.233 | > 0.10 | > 0.10 |
| hsa-miR-107 | 24.85 | 24.49 | 24.65 | 1 | 1.198 | 1.019 | > 0.10 | > 0.10 |
| hsa-miR-10a-3p | 35.86 | 36.47 | 35.21 | 1 | 0.422 | 1.230 | > 0.10 | > 0.10 |
| hsa-miR-10a-5p | 28.77 | 29.63 | 29.59 | 1 | 0.513 | 0.505 | > 0.10 | > 0.10 |
| hsa-miR-10b-3p | 34.39 | 35.96 | 34.33 | 1 | 0.313* | 0.923 | **0.0261** | > 0.10 |
| hsa-miR-10b-5p | 24.98 | 25.69 | 25.00 | 1 | 0.571 | 0.877 | > 0.10 | > 0.10 |
| hsa-miR-1224-3p | 36.23 | 34.01 | 34.56 | 1 | 4.568 | 2.618 | > 0.10 | > 0.10 |
| hsa-miR-122-5p | 32.64 | 35.87 | 35.20 | 1 | 0.094* | 0.133* | **0.0220** | **0.0324** |
| hsa-miR-124-3p | 36.79 | **40.00** | 37.67 | 1 | 0.101** | 0.481 | **0.0012** | > 0.10 |
| hsa-miR-1249 | 35.77 | 33.99 | 33.88 | 1 | 3.103 | 3.377 | > 0.10 | > 0.10 |
| hsa-miR-125a-3p | 32.16 | 31.96 | 31.86 | 1 | 1.067 | 1.092 | > 0.10 | > 0.10 |
| hsa-miR-125a-5p | 23.75 | 23.58 | 23.46 | 1 | 1.048 | 1.088 | > 0.10 | > 0.10 |
| hsa-miR-125b-2-3p | 29.19 | 29.39 | 29.14 | 1 | 0.814 | 0.922 | > 0.10 | > 0.10 |
| hsa-miR-125b-5p | 21.95 | 22.30 | 22.04 | 1 | 0.730 | 0.834 | > 0.10 | > 0.10 |

| hsa-miR-1260a | 22.09 | 21.97 | 22.17 | 1 | 1.016 | 0.846 | > 0.10 | > 0.10 |
|---|---|---|---|---|---|---|---|---|
| hsa-miR-126-3p | 28.52 | 28.58 | 28.19 | 1 | 0.894 | 1.116 | > 0.10 | > 0.10 |
| hsa-miR-126-5p | 33.40 | 33.18 | 33.52 | 1 | 1.083 | 0.817 | > 0.10 | > 0.10 |
| hsa-miR-1267 | 35.62 | 35.66 | 36.20 | 1 | 0.916 | 0.532 | > 0.10 | > 0.10 |
| hsa-miR-1270 | **40.00** | 36.45 | 37.17 | 1 | 10.920 | 6.340 | > 0.10 | > 0.10 |
| hsa-miR-1271-5p | 34.87 | 35.47 | 34.74 | 1 | 0.739 | 0.861 | > 0.10 | > 0.10 |
| hsa-miR-127-3p | 31.52 | 30.93 | 31.78 | 1 | 1.405 | 0.744 | > 0.10 | > 0.10 |
| hsa-miR-128-3p | 30.87 | 30.31 | 30.24 | 1 | 1.380 | 1.376 | > 0.10 | > 0.10 |
| hsa-miR-1296-5p | 31.25 | 31.09 | 30.76 | 1 | 1.039 | 1.244 | > 0.10 | > 0.10 |
| hsa-miR-130a-3p | 32.80 | 32.58 | 32.39 | 1 | 1.082 | 1.176 | > 0.10 | > 0.10 |
| hsa-miR-130b-3p | 32.29 | 32.11 | 32.25 | 1 | 0.919 | 0.913 | > 0.10 | > 0.10 |
| hsa-miR-130b-5p | 32.34 | 32.58 | 33.01 | 1 | 0.791 | 0.560 | > 0.10 | > 0.10 |
| hsa-miR-132-3p | 27.29 | 27.69 | 27.58 | 1 | 0.706 | 0.725 | > 0.10 | > 0.10 |
| hsa-miR-132-5p | 32.32 | 32.79 | 33.28 | 1 | 0.674 | *0.456** | > 0.10 | **0.0484** |
| hsa-miR-134-5p | 34.53 | 33.33 | 33.84 | 1 | 2.152 | 1.440 | > 0.10 | > 0.10 |
| hsa-miR-135a-5p | 23.77 | 23.73 | 23.77 | 1 | 0.954 | 0.885 | > 0.10 | > 0.10 |
| hsa-miR-135b-5p | 29.37 | 31.07 | 30.37 | 1 | 0.288 | 0.446 | 0.0703 | > 0.10 |
| hsa-miR-136-3p | 34.55 | 33.85 | 34.22 | 1 | 1.523 | 0.990 | > 0.10 | > 0.10 |
| hsa-miR-136-5p | 33.15 | 31.44 | 32.37 | 1 | 3.053 | 1.526 | > 0.10 | > 0.10 |
| hsa-miR-139-5p | 35.31 | 36.26 | 35.73 | 1 | 0.484 | 0.667 | > 0.10 | > 0.10 |
| hsa-miR-140-3p | 27.30 | 27.52 | 27.41 | 1 | 0.805 | 0.828 | > 0.10 | > 0.10 |
| hsa-miR-140-5p | 29.75 | 29.64 | 29.45 | 1 | 1.008 | 1.091 | > 0.10 | > 0.10 |
| hsa-miR-141-3p | 21.63 | 21.55 | 21.47 | 1 | 0.990 | 0.998 | > 0.10 | > 0.10 |
| hsa-miR-141-5p | 29.05 | 28.87 | 28.81 | 1 | 1.057 | 1.044 | > 0.10 | > 0.10 |
| hsa-miR-142-3p | 32.00 | 31.71 | 32.66 | 1 | 1.145 | 0.564 | > 0.10 | > 0.10 |
| hsa-miR-142-5p | 36.12 | 36.26 | 35.74 | 1 | 0.833 | 1.460 | > 0.10 | > 0.10 |
| hsa-miR-143-3p | 36.42 | 35.33 | 35.36 | 1 | 1.990 | 1.640 | 0.0861 | > 0.10 |
| hsa-miR-144-3p | 36.18 | 35.52 | 35.08 | 1 | 1.290 | 1.891 | > 0.10 | > 0.10 |
| hsa-miR-145-5p | 35.77 | 36.35 | 35.93 | 1 | 0.594 | 0.895 | > 0.10 | > 0.10 |
| hsa-miR-1468-5p | 32.58 | 32.43 | 32.53 | 1 | 1.037 | 0.918 | > 0.10 | > 0.10 |
| hsa-miR-146a-5p | 29.83 | 28.95 | 29.27 | 1 | 1.794 | 1.369 | > 0.10 | > 0.10 |
| hsa-miR-146b-3p | 33.72 | 35.14 | 35.61 | 1 | 0.348 | 0.240 | 0.0737 | > 0.10 |
| hsa-miR-146b-5p | 29.86 | 31.26 | 31.04 | 1 | 0.355 | 0.393 | 0.0738 | 0.0930 |
| hsa-miR-148a-3p | 23.64 | 22.78 | 22.93 | 1 | 1.708 | 1.465 | > 0.10 | > 0.10 |
| hsa-miR-148b-3p | 27.08 | 26.62 | 26.36 | 1 | 1.279 | 1.464 | > 0.10 | > 0.10 |
| hsa-miR-148b-5p | 32.55 | 32.84 | 32.25 | 1 | 0.761 | 1.095 | > 0.10 | > 0.10 |
| hsa-miR-149-3p | 31.86 | 31.37 | 31.56 | 1 | 1.310 | 1.091 | > 0.10 | > 0.10 |
| hsa-miR-149-5p | 24.69 | 24.51 | 24.43 | 1 | 1.052 | 1.064 | > 0.10 | > 0.10 |
| hsa-miR-150-5p | 32.47 | 33.56 | 33.46 | 1 | 0.438 | 0.448 | 0.0837 | 0.0960 |
| hsa-miR-151a-3p | 26.90 | 26.93 | 26.80 | 1 | 0.913 | 0.953 | > 0.10 | > 0.10 |
| hsa-miR-151a-5p | 24.96 | 25.09 | 24.67 | 1 | 0.856 | 1.090 | > 0.10 | > 0.10 |
| hsa-miR-152-3p | 27.12 | 27.01 | 26.84 | 1 | 1.007 | 1.081 | > 0.10 | > 0.10 |

| | | | | | | | | |
|---|---|---|---|---|---|---|---|---|
| hsa-miR-153-3p | 31.24 | 30.40 | 29.90 | 1 | 1.667 | 2.251* | > 0.10 | **0.0434** |
| hsa-miR-1537-3p | 35.07 | 35.56 | 34.80 | 1 | 0.650 | 1.052 | > 0.10 | > 0.10 |
| hsa-miR-154-5p | 32.68 | 32.22 | 32.95 | 1 | 1.284 | 0.740 | 0.0765 | > 0.10 |
| hsa-miR-155-5p | 26.95 | 27.32 | 27.13 | 1 | 0.722 | 0.782 | > 0.10 | > 0.10 |
| hsa-miR-15a-3p | **38.26** | 35.81 | 37.55 | 1 | 5.112 | 1.456 | > 0.10 | > 0.10 |
| hsa-miR-15a-5p | 25.62 | 25.43 | 25.66 | 1 | 1.067 | 0.869 | > 0.10 | > 0.10 |
| hsa-miR-15b-3p | 32.61 | 32.56 | 32.95 | 1 | 0.962 | 0.703 | > 0.10 | 0.0684 |
| hsa-miR-15b-5p | 28.23 | 27.64 | 28.06 | 1 | 1.400 | 0.999 | > 0.10 | > 0.10 |
| hsa-miR-16-1-3p | 33.43 | 33.17 | 33.33 | 1 | 1.115 | 0.949 | > 0.10 | > 0.10 |
| hsa-miR-16-2-3p | 33.76 | 33.43 | 33.58 | 1 | 1.175 | 1.007 | > 0.10 | > 0.10 |
| hsa-miR-16-5p | 22.09 | 22.60 | 22.53 | 1 | 0.657 | 0.657 | > 0.10 | > 0.10 |
| hsa-miR-17-3p | 31.37 | 30.40 | 30.30 | 1 | 1.829 | 1.869 | > 0.10 | > 0.10 |
| hsa-miR-17-5p | 29.84 | 29.46 | 29.44 | 1 | 1.217 | 1.176 | > 0.10 | > 0.10 |
| hsa-miR-181a-2-3p | 33.45 | 34.02 | 34.47 | 1 | 0.628 | 0.436 | > 0.10 | > 0.10 |
| hsa-miR-181a-5p | 28.56 | 30.24 | 29.52 | 1 | *0.292** | 0.459 | **0.0451** | > 0.10 |
| hsa-miR-181b-5p | 31.40 | 33.32 | 32.70 | 1 | *0.246** | 0.362 | **0.0429** | 0.0907 |
| hsa-miR-181c-3p | 32.96 | 32.47 | 33.24 | 1 | 1.310 | 0.734 | > 0.10 | > 0.10 |
| hsa-miR-181c-5p | 33.58 | 33.65 | 34.18 | 1 | 0.901 | 0.595 | > 0.10 | > 0.10 |
| hsa-miR-181d-5p | 33.67 | 34.96 | 34.81 | 1 | 0.389 | 0.411 | > 0.10 | 0.0981 |
| hsa-miR-182-3p | 34.10 | 35.33 | 33.39 | 1 | *0.399** | 1.454 | **0.0344** | > 0.10 |
| hsa-miR-182-5p | 26.21 | 26.31 | 25.95 | 1 | 0.868 | 1.064 | > 0.10 | > 0.10 |
| hsa-miR-183-3p | 33.75 | 34.29 | 33.86 | 1 | 0.441 | 0.929 | > 0.10 | > 0.10 |
| hsa-miR-183-5p | 27.42 | 27.55 | 27.19 | 1 | 0.851 | 1.045 | > 0.10 | > 0.10 |
| hsa-miR-185-5p | 26.27 | 26.45 | 26.17 | 1 | 0.823 | 0.950 | > 0.10 | > 0.10 |
| hsa-miR-186-5p | 30.24 | 29.94 | 30.18 | 1 | 1.150 | 0.929 | > 0.10 | > 0.10 |
| hsa-miR-187-3p | 28.46 | 28.74 | 28.21 | 1 | 0.769 | 1.057 | > 0.10 | > 0.10 |
| hsa-miR-187-5p | 35.59 | 35.37 | 35.24 | 1 | 0.949 | 1.137 | > 0.10 | > 0.10 |
| hsa-miR-188-3p[4cX] | 35.32 | 33.98 | 34.24 | 1 | 2.012 | 1.846 | > 0.10 | > 0.10 |
| hsa-miR-188-5p[4cX] | 34.53 | 34.86 | 34.71 | 1 | 0.928 | 0.819 | > 0.10 | > 0.10 |
| hsa-miR-18a-3p | 33.07 | 33.05 | 32.72 | 1 | 0.948 | 1.134 | > 0.10 | > 0.10 |
| hsa-miR-18a-5p | 29.20 | 28.78 | 28.74 | 1 | 1.247 | 1.222 | > 0.10 | > 0.10 |
| hsa-miR-18b-3p[5cX] | 34.62 | 34.16 | 33.71 | 1 | 1.290 | 1.673 | > 0.10 | > 0.10 |
| hsa-miR-18b-5p[5cX] | 28.71 | 28.32 | 28.31 | 1 | 1.225 | 1.176 | > 0.10 | > 0.10 |
| hsa-miR-1908-5p | 34.42 | 33.67 | 34.04 | 1 | 1.569 | 1.165 | > 0.10 | > 0.10 |
| hsa-miR-190a-5p | 32.63 | 32.15 | 32.07 | 1 | 1.303 | 1.314 | > 0.10 | > 0.10 |
| hsa-miR-190b | 35.94 | 37.88 | 36.53 | 1 | 0.243 | 0.588 | > 0.10 | > 0.10 |
| hsa-miR-191-3p | 34.01 | 34.35 | 33.68 | 1 | 0.734 | 1.117 | > 0.10 | > 0.10 |
| hsa-miR-191-5p | 24.54 | 24.73 | 24.36 | 1 | 0.817 | 1.008 | > 0.10 | > 0.10 |
| hsa-miR-192-3p | 35.30 | 36.02 | 34.38 | 1 | 0.567 | 1.689 | > 0.10 | > 0.10 |
| hsa-miR-192-5p | 28.23 | 28.77 | 28.44 | 1 | 0.641 | 0.769 | > 0.10 | 0.0598 |

| | | | | | | | | |
|---|---|---|---|---|---|---|---|---|
| hsa-miR-193a-3p | 35.00 | 33.66 | 34.02 | 1 | 2.879* | 1.783 | 0.0424 | > 0.10 |
| hsa-miR-193a-5p | 29.34 | 29.19 | 28.95 | 1 | 1.031 | 1.164 | > 0.10 | > 0.10 |
| hsa-miR-193b-3p | 26.13 | 25.77 | 25.83 | 1 | 1.196 | 1.096 | > 0.10 | > 0.10 |
| hsa-miR-193b-5p | 31.49 | 31.31 | 31.39 | 1 | 1.056 | 0.952 | > 0.10 | > 0.10 |
| hsa-miR-194-5p | 28.10 | 28.59 | 28.18 | 1 | 0.665 | 0.839 | > 0.10 | > 0.10 |
| hsa-miR-195-3p | 34.44 | 34.21 | 34.48 | 1 | 1.091 | 0.865 | > 0.10 | > 0.10 |
| hsa-miR-195-5p | 25.54 | 25.56 | 25.82 | 1 | 0.916 | 0.734 | > 0.10 | > 0.10 |
| hsa-miR-196a-5p | 34.78 | 34.09 | 34.78 | 1 | 1.498 | 0.890 | > 0.10 | > 0.10 |
| hsa-miR-196b-3p | 30.82 | 30.56 | 30.12 | 1 | 1.123 | 1.451* | > 0.10 | 0.0463 |
| hsa-miR-196b-5p | 25.79 | 25.79 | 25.50 | 1 | 0.931 | 1.082 | > 0.10 | > 0.10 |
| hsa-miR-1972 | 32.34 | 32.46 | 33.20 | 1 | 0.853 | 0.489 | > 0.10 | > 0.10 |
| hsa-miR-197-3p | 28.24 | 27.80 | 27.94 | 1 | 1.264 | 1.095 | > 0.10 | > 0.10 |
| hsa-miR-199a-3p | 34.82 | 35.33 | 35.18 | 1 | 0.657 | 0.615 | > 0.10 | > 0.10 |
| hsa-miR-199b-5p | 36.39 | 35.74 | 35.06 | 1 | 1.476 | 1.994 | > 0.10 | > 0.10 |
| hsa-miR-19a-3p | 25.34 | 25.15 | 25.44 | 1 | 1.065 | 0.831 | > 0.10 | > 0.10 |
| hsa-miR-19b-1-5p | 32.35 | 32.30 | 31.98 | 1 | 0.967 | 1.146 | > 0.10 | > 0.10 |
| hsa-miR-19b-2-5p$^{5cX}$ | 35.94 | 35.13 | 35.63 | 1 | 1.423 | 1.101 | > 0.10 | > 0.10 |
| hsa-miR-19b-3p$^{5cX}$ | 22.73 | 22.53 | 22.61 | 1 | 1.067 | 0.963 | > 0.10 | > 0.10 |
| hsa-miR-200a-3p | 23.76 | 23.81 | 23.49 | 1 | 0.902 | 1.070 | > 0.10 | > 0.10 |
| hsa-miR-200a-5p | 29.74 | 29.95 | 29.61 | 1 | 0.804 | 0.972 | > 0.10 | > 0.10 |
| hsa-miR-200b-3p | 22.09 | 22.23 | 21.84 | 1 | 0.849 | 1.058 | 0.0888 | > 0.10 |
| hsa-miR-200b-5p | 27.26 | 27.49 | 27.07 | 1 | 0.798 | 1.017 | > 0.10 | > 0.10 |
| hsa-miR-200c-3p | 20.38 | 20.64 | 20.29 | 1 | 0.782 | 0.950 | > 0.10 | > 0.10 |
| hsa-miR-200c-5p | 31.74 | 31.89 | 31.49 | 1 | 0.842 | 1.053 | > 0.10 | > 0.10 |
| hsa-miR-202-3p | 30.69 | **40.00** | **40.00** | 1 | *0.001** | *0.001** | 0.0004 | 0.0009 |
| hsa-miR-202-5p | 32.18 | **40.00** | **40.00** | 1 | *0.004** | *0.004** | 0.0001 | 0.0001 |
| hsa-miR-203a | 25.16 | 25.07 | 25.18 | 1 | 0.995 | 0.880* | > 0.10 | 0.0377 |
| hsa-miR-204-5p | 29.68 | 29.88 | 30.31 | 1 | 0.810 | 0.575 | > 0.10 | > 0.10 |
| hsa-miR-205-5p | 25.27 | 26.62 | 25.55 | 1 | 0.367* | 0.734 | 0.0257 | > 0.10 |
| hsa-miR-20a-3p | 29.12 | 28.85 | 28.83 | 1 | 1.127 | 1.085 | > 0.10 | > 0.10 |
| hsa-miR-20a-5p | 23.33 | 23.26 | 23.25 | 1 | 0.984 | 0.945 | > 0.10 | > 0.10 |
| hsa-miR-20b-3p$^{5cX}$ | 30.08 | 29.69 | 29.95 | 1 | 1.228 | 0.974 | > 0.10 | > 0.10 |
| hsa-miR-20b-5p$^{5cX}$ | 28.19 | 27.50 | 27.94 | 1 | 1.503 | 1.059 | > 0.10 | > 0.10 |
| hsa-miR-210-3p | 27.96 | 28.00 | 27.58 | 1 | 0.907 | 1.154 | > 0.10 | > 0.10 |
| hsa-miR-2110 | 30.83 | 31.04 | 30.47 | 1 | 0.803 | 1.135 | > 0.10 | > 0.10 |
| hsa-miR-212-3p | 32.51 | 32.15 | 32.72 | 1 | 1.199 | 0.770 | > 0.10 | > 0.10 |
| hsa-miR-212-5p | 35.93 | 35.76 | 35.71 | 1 | 1.054 | 0.919 | > 0.10 | > 0.10 |
| hsa-miR-21-3p | 33.43 | 32.19 | 32.69 | 1 | 2.202* | 1.485 | 0.0372 | > 0.10 |
| hsa-miR-215-5p | 29.66 | 30.21 | 29.81 | 1 | 0.639 | 0.801 | 0.0579 | > 0.10 |
| hsa-miR-21-5p | 21.09 | 20.64 | 20.86 | 1 | 1.270 | 1.047 | > 0.10 | > 0.10 |

| | | | | | | | | |
|---|---|---|---|---|---|---|---|---|
| hsa-miR-217 | 36.64 | 35.61 | 35.85 | 1 | 2.504 | 1.547 | > 0.10 | > 0.10 |
| hsa-miR-218-5p | 31.89 | 32.04 | 31.48 | 1 | 0.836 | 1.175 | > 0.10 | > 0.10 |
| hsa-miR-219a-5p | 31.98 | 31.49 | 31.72 | 1 | 1.308 | 1.067 | > 0.10 | > 0.10 |
| hsa-miR-221-3p | 28.84 | 30.30 | 29.69 | 1 | 0.338 | 0.492 | > 0.10 | > 0.10 |
| hsa-miR-222-3p | 26.18 | 27.71 | 26.82 | 1 | 0.323* | 0.570 | 0.0296 | > 0.10 |
| hsa-miR-223-3p | 31.17 | 29.89 | 31.25 | 1 | 2.263 | 0.838 | > 0.10 | > 0.10 |
| hsa-miR-22-3p | 27.52 | 26.77 | 26.90 | 1 | 1.562 | 1.361 | > 0.10 | > 0.10 |
| hsa-miR-224-3p | 33.96 | 34.98 | 36.27 | 1 | 0.436 | 0.202 | > 0.10 | 0.0818 |
| hsa-miR-224-5p | 35.31 | 35.69 | 36.03 | 1 | 0.680 | 0.477 | > 0.10 | > 0.10 |
| hsa-miR-22-5p | 29.60 | 28.84 | 28.90 | 1 | 1.581 | 1.445 | > 0.10 | > 0.10 |
| hsa-miR-23a-3p | 23.90 | 24.04 | 24.02 | 1 | 0.847 | 0.823 | > 0.10 | > 0.10 |
| hsa-miR-23b-3p | 23.56 | 23.63 | 23.50 | 1 | 0.888 | 0.928 | > 0.10 | > 0.10 |
| hsa-miR-23b-5p | 35.46 | 33.99 | 34.71 | 1 | 2.590* | 1.498 | 0.0284 | > 0.10 |
| hsa-miR-24-1-5p | 33.69 | 33.18 | 33.04 | 1 | 1.325 | 1.390 | > 0.10 | > 0.10 |
| hsa-miR-24-2-5p | 32.74 | 32.67 | 32.72 | 1 | 0.976 | 0.900 | > 0.10 | > 0.10 |
| hsa-miR-24-3p | 23.67 | 23.89 | 23.61 | 1 | 0.805 | 0.929 | > 0.10 | > 0.10 |
| hsa-miR-25-3p | 25.83 | 26.01 | 25.59 | 1 | 0.824 | 1.054 | > 0.10 | > 0.10 |
| hsa-miR-25-5p | 33.68 | 33.57 | 33.66 | 1 | 1.012 | 0.904 | > 0.10 | > 0.10 |
| hsa-miR-26a-1-3p | 33.96 | 33.84 | 33.54 | 1 | 1.017 | 1.197 | > 0.10 | > 0.10 |
| hsa-miR-26a-2-3p | 36.20 | 36.81 | 36.07 | 1 | 0.679 | 0.981 | > 0.10 | > 0.10 |
| hsa-miR-26a-5p | 23.23 | 23.19 | 23.18 | 1 | 0.958 | 0.919 | > 0.10 | > 0.10 |
| hsa-miR-26b-3p | 32.01 | 31.57 | 31.50 | 1 | 1.258 | 1.261 | > 0.10 | > 0.10 |
| hsa-miR-26b-5p | 24.94 | 24.20 | 24.31 | 1 | 1.560 | 1.374 | > 0.10 | > 0.10 |
| hsa-miR-27a-3p | 27.70 | 27.17 | 27.37 | 1 | 1.346 | 1.119 | > 0.10 | > 0.10 |
| hsa-miR-27b-3p | 24.82 | 24.77 | 24.68 | 1 | 0.964 | 0.975 | > 0.10 | > 0.10 |
| hsa-miR-27b-5p | 33.11 | 33.26 | 32.94 | 1 | 0.836 | 1.000 | > 0.10 | > 0.10 |
| hsa-miR-28-3p | 26.70 | 26.79 | 26.67 | 1 | 0.876 | 0.912 | > 0.10 | > 0.10 |
| hsa-miR-28-5p | 26.33 | 26.31 | 26.17 | 1 | 0.943 | 0.990 | > 0.10 | > 0.10 |
| hsa-miR-296-3p | 35.52 | 35.77 | 36.13 | 1 | 0.795 | 0.667 | > 0.10 | > 0.10 |
| hsa-miR-296-5p | 34.74 | 32.75 | 33.48 | 1 | 3.705* | 2.400 | 0.0177 | > 0.10 |
| hsa-miR-29a-3p | 26.48 | 25.68 | 25.94 | 1 | 1.614* | 1.288 | 0.0103 | > 0.10 |
| hsa-miR-29a-5p | 28.35 | 28.26 | 28.32 | 1 | 0.989 | 0.907 | > 0.10 | > 0.10 |
| hsa-miR-29b-2-5p | 28.90 | 28.87 | 28.63 | 1 | 0.951 | 1.072 | > 0.10 | > 0.10 |
| hsa-miR-29b-3p | 25.73 | 25.03 | 24.96 | 1 | 1.517 | 1.516 | > 0.10 | > 0.10 |
| hsa-miR-29c-3p | 25.29 | 24.43 | 24.62 | 1 | 1.687* | 1.415 | 0.0468 | > 0.10 |
| hsa-miR-29c-5p | 30.81 | 30.04 | 30.08 | 1 | 1.582 | 1.466 | 0.0759 | > 0.10 |
| hsa-miR-301a-3p | 30.31 | 30.63 | 30.23 | 1 | 0.748 | 0.941 | > 0.10 | > 0.10 |
| hsa-miR-301b | 33.65 | 34.40 | 33.68 | 1 | 0.556 | 0.872 | > 0.10 | > 0.10 |
| hsa-miR-30a-3p | 27.93 | 27.83 | 27.94 | 1 | 1.003 | 0.887 | > 0.10 | > 0.10 |
| hsa-miR-30a-5p | 26.08 | 25.73 | 26.27 | 1 | 1.193 | 0.781 | > 0.10 | > 0.10 |
| hsa-miR-30b-3p | 35.80 | 34.66 | 34.51 | 1 | 2.050 | 2.174 | 0.0914 | > 0.10 |

| | | | | | | | | |
|---|---|---|---|---|---|---|---|---|
| hsa-miR-30b-5p | 23.30 | 23.31 | 23.31 | 1 | 0.929 | 0.886 | > 0.10 | > 0.10 |
| hsa-miR-30c-1-3p | 35.63 | 35.69 | 35.09 | 1 | 0.895 | 1.290 | > 0.10 | > 0.10 |
| hsa-miR-30c-5p | 23.93 | 23.83 | 23.88 | 1 | 1.001 | 0.919 | > 0.10 | 0.0630 |
| hsa-miR-30d-3p | 30.36 | 30.09 | 30.21 | 1 | 1.126 | 0.986 | > 0.10 | > 0.10 |
| hsa-miR-30d-5p | 24.02 | 23.97 | 23.87 | 1 | 0.969 | 0.985 | > 0.10 | > 0.10 |
| hsa-miR-30e-3p | 26.73 | 26.71 | 26.56 | 1 | 0.950 | 1.005 | > 0.10 | > 0.10 |
| hsa-miR-30e-5p | 24.65 | 24.72 | 24.59 | 1 | 0.889 | 0.930 | > 0.10 | > 0.10 |
| hsa-miR-31-3p | 32.20 | 34.10 | 33.48 | 1 | 0.250* | 0.367 | 0.0481 | > 0.10 |
| hsa-miR-31-5p | 28.68 | 30.31 | 29.38 | 1 | 0.302* | 0.548 | 0.0387 | > 0.10 |
| hsa-miR-320a | 25.21 | 25.43 | 24.96 | 1 | 0.800 | 1.056 | > 0.10 | > 0.10 |
| hsa-miR-320b | 26.60 | 26.88 | 26.45 | 1 | 0.765 | 0.985 | > 0.10 | > 0.10 |
| hsa-miR-320c | 28.35 | 28.28 | 27.77 | 1 | 0.982 | 1.332 | > 0.10 | > 0.10 |
| hsa-miR-320d | 29.69 | 30.22 | 29.33 | 1 | 0.778 | 1.140 | > 0.10 | > 0.10 |
| hsa-miR-323a-3p | 34.64 | 33.46 | 34.91 | 1 | 2.118 | 0.738 | > 0.10 | > 0.10 |
| hsa-miR-32-3p | 34.03 | 33.81 | 33.41 | 1 | 1.087 | 1.370 | > 0.10 | > 0.10 |
| hsa-miR-324-3p | 27.77 | 27.36 | 27.39 | 1 | 1.240 | 1.155 | > 0.10 | > 0.10 |
| hsa-miR-324-5p | 27.27 | 26.96 | 26.80 | 1 | 1.158 | 1.229 | > 0.10 | > 0.10 |
| hsa-miR-32-5p | 28.59 | 27.84 | 28.21 | 1 | 1.566 | 1.159 | > 0.10 | > 0.10 |
| hsa-miR-328-3p | 29.90 | 29.49 | 29.51 | 1 | 1.235 | 1.162 | > 0.10 | > 0.10 |
| hsa-miR-330-3p | 32.73 | 32.59 | 32.44 | 1 | 1.025 | 1.087 | > 0.10 | > 0.10 |
| hsa-miR-330-5p | 35.10 | 35.29 | 33.79 | 1 | 0.797 | 1.700 | > 0.10 | > 0.10 |
| hsa-miR-331-3p | 27.63 | 27.69 | 27.66 | 1 | 0.898 | 0.869 | > 0.10 | > 0.10 |
| hsa-miR-331-5p | 34.97 | 35.24 | 35.25 | 1 | 0.781 | 0.737 | > 0.10 | > 0.10 |
| hsa-miR-335-3p | 34.12 | 35.46 | 34.59 | 1 | 0.369 | 0.641 | 0.0529 | > 0.10 |
| hsa-miR-335-5p | 30.14 | 30.44 | 29.86 | 1 | 0.756 | 1.081 | > 0.10 | > 0.10 |
| hsa-miR-338-3p | 29.30 | 28.74 | 29.00 | 1 | 1.373 | 1.093 | > 0.10 | > 0.10 |
| hsa-miR-338-5p | 35.91 | 36.48 | 36.76 | 1 | 0.653 | 0.454 | > 0.10 | > 0.10 |
| hsa-miR-339-3p | 29.34 | 29.20 | 29.25 | 1 | 1.028 | 0.949 | > 0.10 | > 0.10 |
| hsa-miR-339-5p | 28.16 | 27.90 | 27.93 | 1 | 1.118 | 1.042 | > 0.10 | > 0.10 |
| hsa-miR-33a-3p | 30.87 | 30.65 | 30.64 | 1 | 1.087 | 1.045 | > 0.10 | > 0.10 |
| hsa-miR-33a-5p | 29.26 | 27.83 | 28.36 | 1 | 2.528 | 1.663 | 0.0928 | > 0.10 |
| hsa-miR-33b-3p | 31.94 | 31.87 | 32.44 | 1 | 0.976 | 0.628 | > 0.10 | > 0.10 |
| hsa-miR-33b-5p | 35.06 | 33.59 | 34.20 | 1 | 2.583 | 1.613 | 0.0968 | > 0.10 |
| hsa-miR-340-3p | 32.34 | 32.06 | 31.64 | 1 | 1.131 | 1.440 | > 0.10 | 0.0699 |
| hsa-miR-340-5p | 30.47 | 30.29 | 30.34 | 1 | 0.960 | 0.893 | > 0.10 | > 0.10 |
| hsa-miR-342-3p | 25.37 | 25.65 | 25.54 | 1 | 0.772 | 0.790 | > 0.10 | > 0.10 |
| hsa-miR-342-5p | 33.01 | 32.96 | 33.15 | 1 | 0.840 | 0.809 | > 0.10 | > 0.10 |
| hsa-miR-345-5p | 27.66 | 27.79 | 27.48 | 1 | 0.852 | 1.009 | > 0.10 | > 0.10 |
| hsa-miR-346 | 35.04 | 34.86 | 36.03 | 1 | 1.284 | 0.454 | > 0.10 | > 0.10 |
| hsa-miR-34a-3p | 31.32 | 31.05 | 30.39 | 1 | 1.131 | 1.704 | > 0.10 | > 0.10 |
| hsa-miR-34a-5p | 28.28 | 27.70 | 26.59 | 1 | 1.393 | 2.862 | > 0.10 | 0.0665 |
| hsa-miR-34b-3p | 32.35 | 35.66 | 35.28 | 1 | 0.124 | 0.132 | > 0.10 | > 0.10 |

24

| | | | | | | | | |
|---|---|---|---|---|---|---|---|---|
| hsa-miR-34b-5p | 31.44 | 35.21 | 33.86 | 1 | 0.007* | 0.167* | 0.0272 | 0.0163 |
| hsa-miR-34c-3p | 33.83 | 36.96 | 35.06 | 1 | 0.093* | 0.382 | 0.0087 | > 0.10 |
| hsa-miR-34c-5p | 30.18 | 34.17 | 33.78 | 1 | 0.059** | 0.073* | 0.0005 | 0.0244 |
| hsa-miR-361-3p | 30.59 | 29.69 | 29.77 | 1 | 1.741* | 1.575 | 0.0473 | 0.0720 |
| hsa-miR-361-5p | 25.45 | 25.37 | 25.20 | 1 | 0.986 | 1.053 | > 0.10 | > 0.10 |
| hsa-miR-362-3p[4cX] | 29.95 | 30.02 | 30.09 | 1 | 0.891 | 0.808 | > 0.10 | > 0.10 |
| hsa-miR-362-5p[4cX] | 32.07 | 32.47 | 32.26 | 1 | 0.708 | 0.781 | > 0.10 | > 0.10 |
| hsa-miR-363-3p[5cX] | 24.42 | 23.76 | 23.94 | 1 | 1.474** | 1.241** | 0.0002 | 0.0043 |
| hsa-miR-363-5p[5cX] | 34.82 | 33.94 | 33.65 | 1 | 1.725 | 2.004 | > 0.10 | > 0.10 |
| hsa-miR-365a-3p | 28.61 | 27.76 | 27.96 | 1 | 1.689* | 1.394* | 0.0350 | 0.0171 |
| hsa-miR-365b-5p | 35.70 | 35.26 | 34.07 | 1 | 1.267 | 2.760 | > 0.10 | > 0.10 |
| hsa-miR-370-3p | 36.90 | 34.64 | 35.83 | 1 | 3.795 | 1.594 | > 0.10 | > 0.10 |
| hsa-miR-374a-5p | 27.93 | 27.48 | 27.70 | 1 | 1.280 | 1.044 | > 0.10 | > 0.10 |
| hsa-miR-374b-3p | 33.70 | 32.65 | 33.03 | 1 | 1.933 | 1.415 | > 0.10 | > 0.10 |
| hsa-miR-374b-5p | 26.44 | 26.31 | 26.30 | 1 | 1.018 | 0.980 | > 0.10 | > 0.10 |
| hsa-miR-375 | 20.85 | 21.15 | 20.63 | 1 | 0.758 | 1.030 | > 0.10 | > 0.10 |
| hsa-miR-376a-3p | 32.77 | 31.84 | 32.78 | 1 | 1.781 | 0.884 | > 0.10 | > 0.10 |
| hsa-miR-376b-3p | 34.83 | 33.93 | 35.20 | 1 | 1.544 | 0.702 | > 0.10 | > 0.10 |
| hsa-miR-376c-3p | 32.35 | 31.31 | 32.54 | 1 | 1.923 | 0.782 | > 0.10 | > 0.10 |
| hsa-miR-378a-5p | 36.44 | 35.74 | 36.12 | 1 | 1.538 | 1.266 | > 0.10 | > 0.10 |
| hsa-miR-379-5p | 34.12 | 32.96 | 34.64 | 1 | 2.071 | 0.618 | > 0.10 | > 0.10 |
| hsa-miR-381-3p | 35.54 | 35.24 | 35.67 | 1 | 1.197 | 0.837 | > 0.10 | > 0.10 |
| hsa-miR-382-5p | 33.25 | 32.75 | 33.79 | 1 | 1.318 | 0.609 | > 0.10 | > 0.10 |
| hsa-miR-383-5p | 36.03 | **40.00** | **40.00** | 1 | 0.059** | 0.057** | 0.0031 | 0.0010 |
| hsa-miR-409-3p | 33.61 | 32.84 | 32.48 | 1 | 1.601 | 1.947 | > 0.10 | > 0.10 |
| hsa-miR-410-3p | 35.92 | 34.35 | 34.96 | 1 | 2.801 | 1.753 | > 0.10 | > 0.10 |
| hsa-miR-411-5p | 33.96 | 33.65 | 34.07 | 1 | 1.156 | 0.826 | > 0.10 | > 0.10 |
| hsa-miR-421 | 29.66 | 30.02 | 30.04 | 1 | 0.725 | 0.685 | > 0.10 | > 0.10 |
| hsa-miR-423-3p | 26.27 | 25.88 | 25.58 | 1 | 1.223 | 1.429 | > 0.10 | 0.0673 |
| hsa-miR-423-5p | 27.59 | 27.31 | 26.85 | 1 | 1.129 | 1.485* | > 0.10 | 0.0165 |
| hsa-miR-424-5p | 30.61 | 32.75 | 31.68 | 1 | 0.212* | 0.424 | 0.0392 | > 0.10 |
| hsa-miR-425-3p | 29.84 | 29.71 | 29.66 | 1 | 1.017 | 1.006 | > 0.10 | > 0.10 |
| hsa-miR-425-5p | 27.73 | 27.60 | 27.48 | 1 | 1.016 | 1.051 | > 0.10 | > 0.10 |
| hsa-miR-429 | 26.61 | 26.33 | 26.34 | 1 | 1.132 | 1.075 | > 0.10 | > 0.10 |
| hsa-miR-432-5p | 35.78 | 34.95 | 35.54 | 1 | 1.664 | 1.050 | > 0.10 | > 0.10 |
| hsa-miR-449a | 30.27 | 32.25 | 32.38 | 1 | 0.237* | 0.205** | 0.0193 | 0.0020 |
| hsa-miR-449b-3p | 35.71 | 36.16 | 35.75 | 1 | 0.594 | 0.852 | > 0.10 | > 0.10 |
| hsa-miR-450a-5p | 34.36 | 36.21 | 35.52 | 1 | 0.339 | 0.399 | > 0.10 | > 0.10 |
| hsa-miR-451a | 35.25 | 34.04 | 34.88 | 1 | 2.184 | 1.158 | > 0.10 | > 0.10 |
| hsa-miR-452-5p | 32.94 | 36.83 | 35.53 | 1 | 0.063* | 0.148* | 0.0164 | 0.0355 |
| hsa-miR-454-3p | 29.58 | 29.65 | 29.45 | 1 | 0.891 | 0.974 | > 0.10 | > 0.10 |

| | | | | | | | | |
|---|---|---|---|---|---|---|---|---|
| hsa-miR-455-3p | 30.52 | 31.88 | 31.54 | 1 | 0.364* | 0.439 | 0.0097 | 0.0750 |
| hsa-miR-455-5p | 33.47 | 34.76 | 35.05 | 1 | 0.382* | 0.297* | 0.0419 | 0.0442 |
| hsa-miR-484 | 28.42 | 28.00 | 28.03 | 1 | 1.253 | 1.162 | > 0.10 | > 0.10 |
| hsa-miR-485-3p | 35.68 | 35.49 | 35.16 | 1 | 1.295 | 1.287 | > 0.10 | > 0.10 |
| hsa-miR-486-5p | 33.60 | 32.18 | 32.79 | 1 | 2.512 | 1.568 | > 0.10 | > 0.10 |
| hsa-miR-487b-3p | 34.15 | 32.96 | 33.78 | 1 | 2.133 | 1.154 | > 0.10 | > 0.10 |
| hsa-miR-491-3p | 35.64 | 34.65 | 35.27 | 1 | 1.279 | 1.149 | > 0.10 | > 0.10 |
| hsa-miR-491-5p | 29.82 | 29.70 | 29.44 | 1 | 1.009 | 1.155 | > 0.10 | > 0.10 |
| hsa-miR-493-3p | 36.58 | 34.18 | 34.20 | 1 | 4.482 | 4.252 | > 0.10 | > 0.10 |
| hsa-miR-493-5p | 36.58 | 35.44 | 35.71 | 1 | 2.073 | 1.622 | 0.0765 | > 0.10 |
| hsa-miR-495-3p | 34.50 | 32.90 | 34.31 | 1 | 2.841 | 1.020 | > 0.10 | > 0.10 |
| hsa-miR-497-5p | 28.10 | 27.31 | 27.45 | 1 | 1.612 | 1.394 | > 0.10 | > 0.10 |
| hsa-miR-499a-5p | 34.44 | 35.88 | 35.93 | 1 | 0.343* | 0.315* | 0.0382 | 0.0310 |
| hsa-miR-500a-5p[4cX] | 30.54 | 31.36 | 31.05 | 1 | 0.530 | 0.624 | > 0.10 | > 0.10 |
| hsa-miR-501-3p[4cX] | 31.72 | 32.13 | 32.04 | 1 | 0.703 | 0.716 | > 0.10 | > 0.10 |
| hsa-miR-501-5p[4cX] | 31.08 | 32.31 | 31.95 | 1 | 0.397 | 0.485 | 0.0677 | > 0.10 |
| hsa-miR-502-3p[4cX] | 30.76 | 31.10 | 30.95 | 1 | 0.739 | 0.779 | > 0.10 | > 0.10 |
| hsa-miR-504-5p | 34.06 | 33.00 | 33.55 | 1 | 1.957 | 1.442 | > 0.10 | > 0.10 |
| hsa-miR-505-3p | 30.15 | 30.27 | 30.02 | 1 | 0.860 | 0.977 | > 0.10 | > 0.10 |
| hsa-miR-505-5p | 34.38 | 34.63 | 33.79 | 1 | 0.785 | 1.331 | > 0.10 | > 0.10 |
| hsa-miR-506-3p[1cX] | 35.77 | **40.00** | **40.00** | 1 | 0.050** | 0.047** | 0.0014 | 0.0003 |
| hsa-miR-508-5p[1cX] | 35.14 | **40.00** | **40.00** | 1 | 0.032** | 0.031** | 0.0029 | 0.0045 |
| hsa-miR-509-3-5p[1cX] | 31.96 | **40.00** | 37.73 | 1 | 0.004** | 0.016* | 0.0000 | 0.0206 |
| hsa-miR-509-3p[1cX] | 32.81 | 34.30 | 34.39 | 1 | 0.318 | 0.299 | 0.0866 | > 0.10 |
| hsa-miR-510-5p[2cX] | 33.14 | **40.00** | **40.00** | 1 | 0.008** | 0.008** | 0.0002 | 0.0000 |
| hsa-miR-513a-3p[1cX] | 35.30 | **40.00** | **40.00** | 1 | 0.036* | 0.034* | 0.0232 | 0.0257 |
| hsa-miR-513b-5p[3cX] | 35.82 | **39.08** | **39.79** | 1 | 0.097 | 0.057 | 0.0629 | 0.0559 |
| hsa-miR-513c-5p[3cX] | 33.42 | **40.00** | **40.00** | 1 | 0.010** | 0.009** | 0.0015 | 0.0025 |
| hsa-miR-514a-3p[2cX] | 29.69 | **40.00** | **40.00** | 1 | 0.001** | 0.001** | 0.0001 | 0.0002 |
| hsa-miR-517a-3p[1c19] | 33.11 | 35.09 | 34.97 | 1 | 0.163 | 0.192 | > 0.10 | > 0.10 |
| hsa-miR-517c-3p[2c19] | 33.39 | 37.14 | 36.67 | 1 | 0.069** | 0.091* | 0.0056 | 0.0285 |
| hsa-miR-518e-3p[1c19] | 34.75 | **39.76** | 38.22 | 1 | 0.029** | 0.080* | 0.0000 | 0.0331 |
| hsa-miR-519d-3p[1c19] | 35.00 | 36.69 | 36.83 | 1 | 0.202 | 0.255 | > 0.10 | > 0.10 |
| hsa-miR-520c-3p[1c19] | 37.10 | **40.00** | **40.00** | 1 | 0.125 | 0.119 | 0.0866 | 0.0867 |
| hsa-miR-520h[2c19] | 35.29 | **40.00** | 39.33 | 1 | 0.036** | 0.054* | 0.0008 | 0.0369 |
| hsa-miR- | 35.57 | **40.00** | **40.00** | 1 | 0.042* | 0.040* | 0.0201 | 0.0317 |

521[1c19.2c19]

| | | | | | | | | |
|---|---|---|---|---|---|---|---|---|
| hsa-miR-532-3p[4cX] | 27.90 | 28.05 | 27.84 | 1 | 0.839 | 0.930 | > 0.10 | > 0.10 |
| hsa-miR-532-5p[4cX] | 28.04 | 28.44 | 28.13 | 1 | 0.708 | 0.839 | > 0.10 | > 0.10 |
| hsa-miR-539-5p | 34.29 | 33.18 | 34.56 | 1 | 2.006 | 0.735 | > 0.10 | > 0.10 |
| hsa-miR-543 | 35.25 | 33.30 | 33.93 | 1 | 3.602 | 2.223 | 0.0570 | > 0.10 |
| hsa-miR-545-3p | 32.79 | 32.56 | 32.32 | 1 | 1.095 | 1.231 | > 0.10 | > 0.10 |
| hsa-miR-548b-3p | 36.55 | 36.74 | 36.07 | 1 | 0.589 | 1.289 | > 0.10 | > 0.10 |
| hsa-miR-548j-5p | 35.25 | 36.05 | 36.26 | 1 | 0.706 | 0.436 | > 0.10 | > 0.10 |
| hsa-miR-548k | 35.08 | 35.07 | 34.33 | 1 | 0.945 | 1.505 | > 0.10 | > 0.10 |
| hsa-miR-548l | 36.40 | 36.65 | 36.31 | 1 | 1.028 | 1.068 | > 0.10 | > 0.10 |
| hsa-miR-550a-3p | 35.75 | 35.72 | 35.48 | 1 | 0.957 | 1.076 | > 0.10 | > 0.10 |
| hsa-miR-550a-5p | **40.00** | **38.60** | 36.59 | 1 | 2.465 | 9.455* | > 0.10 | **0.0094** |
| hsa-miR-551b-3p | 33.44 | 36.18 | 35.29 | 1 | *0.139*** | 0.246 | **0.0042** | 0.0764 |
| hsa-miR-570-3p | 33.46 | 33.70 | 33.19 | 1 | 0.797 | 1.087 | > 0.10 | > 0.10 |
| hsa-miR-574-3p | 24.42 | 24.64 | 24.27 | 1 | 0.803 | 0.988 | > 0.10 | > 0.10 |
| hsa-miR-576-3p | 35.09 | 34.82 | 35.27 | 1 | 0.778 | 0.892 | > 0.10 | > 0.10 |
| hsa-miR-576-5p | 34.93 | 34.31 | 34.00 | 1 | 1.434 | 1.697 | > 0.10 | > 0.10 |
| hsa-miR-582-3p | 32.15 | 32.15 | 32.01 | 1 | 0.936 | 1.108 | > 0.10 | > 0.10 |
| hsa-miR-582-5p | 27.62 | 27.70 | 27.60 | 1 | 0.883 | 0.903 | > 0.10 | > 0.10 |
| hsa-miR-589-3p | 33.05 | 33.15 | 33.02 | 1 | 0.872 | 0.911 | > 0.10 | > 0.10 |
| hsa-miR-589-5p | 34.38 | 33.51 | 33.59 | 1 | 1.698 | 1.531 | > 0.10 | > 0.10 |
| hsa-miR-590-3p | 31.33 | 31.06 | 30.88 | 1 | 1.119 | 1.212 | > 0.10 | > 0.10 |
| hsa-miR-590-5p | 27.59 | 27.66 | 27.47 | 1 | 0.887 | 0.970 | > 0.10 | > 0.10 |
| hsa-miR-592 | 35.37 | 34.81 | 34.93 | 1 | 1.370 | 1.208 | > 0.10 | > 0.10 |
| hsa-miR-598-3p | 29.03 | 29.22 | 28.68 | 1 | 0.817 | 1.127 | > 0.10 | > 0.10 |
| hsa-miR-616-3p | 35.47 | 36.06 | 36.31 | 1 | 0.427 | 0.491 | > 0.10 | > 0.10 |
| hsa-miR-616-5p | 35.14 | 35.04 | 35.65 | 1 | 1.001 | 0.625 | > 0.10 | > 0.10 |
| hsa-miR-618 | 36.35 | 35.84 | 35.20 | 1 | 1.613 | 1.562 | > 0.10 | > 0.10 |
| hsa-miR-625-3p | 34.95 | 34.64 | 35.16 | 1 | 1.160 | 0.769 | > 0.10 | > 0.10 |
| hsa-miR-627-5p | 33.89 | 33.47 | 34.04 | 1 | 1.244 | 0.798 | > 0.10 | > 0.10 |
| hsa-miR-628-3p | 32.30 | 32.78 | 32.78 | 1 | 0.667 | 0.636 | > 0.10 | > 0.10 |
| hsa-miR-628-5p | 33.28 | 33.80 | 33.60 | 1 | 0.650 | 0.713 | > 0.10 | > 0.10 |
| hsa-miR-629-3p | 33.73 | 33.07 | 33.38 | 1 | 1.469 | 1.136 | > 0.10 | > 0.10 |
| hsa-miR-629-5p | 30.75 | 30.99 | 30.60 | 1 | 0.789 | 0.989 | > 0.10 | > 0.10 |
| hsa-miR-632 | 35.99 | 34.85 | 35.54 | 1 | 2.074 | 1.211 | 0.0923 | > 0.10 |
| hsa-miR-641 | 37.58 | 36.35 | 37.36 | 1 | 2.189 | 1.037 | > 0.10 | > 0.10 |
| hsa-miR-642a-5p | 36.22 | 35.99 | 35.43 | 1 | 1.094 | 1.541 | > 0.10 | > 0.10 |
| hsa-miR-651-5p | 33.88 | 33.41 | 33.70 | 1 | 1.295 | 1.012 | > 0.10 | > 0.10 |
| hsa-miR-652-3p | 28.36 | 27.79 | 27.73 | 1 | 1.382 | 1.374 | > 0.10 | > 0.10 |
| hsa-miR-660-5p[4cX] | 27.23 | 27.50 | 27.25 | 1 | 0.775 | 0.875 | > 0.10 | > 0.10 |
| hsa-miR-663a | 30.95 | 30.90 | 30.84 | 1 | 0.963 | 0.960 | > 0.10 | > 0.10 |

| | | | | | | | | |
|---|---|---|---|---|---|---|---|---|
| hsa-miR-664a-3p | 29.92 | 29.91 | 30.01 | 1 | 0.937 | 0.837 | > 0.10 | > 0.10 |
| hsa-miR-671-3p | 32.06 | 32.29 | 32.06 | 1 | 0.795 | 0.891 | 0.0785 | > 0.10 |
| hsa-miR-671-5p | 33.68 | 33.41 | 34.00 | 1 | 1.130 | 0.715 | > 0.10 | > 0.10 |
| hsa-miR-708-5p | 33.84 | 35.32 | 33.17 | 1 | 0.332 | 1.409 | > 0.10 | > 0.10 |
| hsa-miR-7-1-3p | 28.72 | 28.90 | 28.71 | 1 | 0.824 | 0.898 | > 0.10 | > 0.10 |
| hsa-miR-744-3p | 32.83 | 32.98 | 32.47 | 1 | 0.843 | 1.141 | > 0.10 | > 0.10 |
| hsa-miR-744-5p | 29.88 | 29.71 | 29.59 | 1 | 1.048 | 1.088 | > 0.10 | > 0.10 |
| hsa-miR-7-5p | 31.11 | 30.85 | 30.43 | 1 | 1.127 | 1.436 | > 0.10 | > 0.10 |
| hsa-miR-766-3p | 30.72 | 30.88 | 30.15 | 1 | 0.833 | 1.321 | > 0.10 | > 0.10 |
| hsa-miR-769-5p | 30.76 | 30.45 | 30.45 | 1 | 1.154 | 1.106 | > 0.10 | > 0.10 |
| hsa-miR-873-5p | 36.50 | **40.00** | **40.00** | 1 | *0.082\*\** | *0.079\*\** | **0.0026** | **0.0037** |
| hsa-miR-877-3p | 36.07 | 35.96 | 36.62 | 1 | 0.859 | 0.596 | > 0.10 | > 0.10 |
| hsa-miR-877-5p | 32.02 | 32.15 | 32.09 | 1 | 0.744 | 0.847 | > 0.10 | > 0.10 |
| hsa-miR-885-3p | 34.13 | 34.19 | 34.43 | 1 | 0.831 | 0.708 | > 0.10 | > 0.10 |
| hsa-miR-887-3p | 35.66 | 35.50 | 34.37 | 1 | 0.719 | 2.149 | > 0.10 | > 0.10 |
| hsa-miR-888-5p[6cX] | 25.93 | 29.18 | 27.11 | 1 | *0.098\** | 0.391 | **0.0324** | > 0.10 |
| hsa-miR-890[6cX] | 26.85 | 30.22 | 28.14 | 1 | *0.090\** | 0.363 | **0.0235** | 0.0590 |
| hsa-miR-891a-5p | 24.00 | 27.26 | 24.99 | 1 | *0.097\** | 0.448 | **0.0266** | > 0.10 |
| hsa-miR-891b[6cX] | 28.56 | 32.03 | 29.53 | 1 | *0.084\** | 0.455 | **0.0387** | > 0.10 |
| hsa-miR-892a[6cX] | 29.42 | 32.54 | 30.13 | 1 | *0.107\** | 0.544 | **0.0148** | > 0.10 |
| hsa-miR-92a-1-5p | 34.24 | 33.57 | 33.28 | 1 | 1.486 | 1.734 | > 0.10 | 0.0570 |
| hsa-miR-92a-2-5p[5cX] | 32.45 | 31.54 | 32.02 | 1 | 1.762 | 1.202 | > 0.10 | > 0.10 |
| hsa-miR-92a-3p[5cX] | 24.06 | 23.70 | 23.79 | 1 | 1.194 | 1.074 | > 0.10 | > 0.10 |
| hsa-miR-92b-3p | 34.30 | 33.58 | 34.19 | 1 | 1.533 | 0.959 | > 0.10 | > 0.10 |
| hsa-miR-93-3p | 29.70 | 29.46 | 29.38 | 1 | 1.095 | 1.107 | > 0.10 | > 0.10 |
| hsa-miR-934 | 34.82 | 34.41 | 35.63 | 1 | 1.510 | 0.514 | > 0.10 | 0.0857 |
| hsa-miR-935 | **39.07** | 37.75 | 37.12 | 1 | 2.317 | 3.436 | > 0.10 | > 0.10 |
| hsa-miR-93-5p | 24.61 | 24.71 | 24.30 | 1 | 0.872 | 1.102 | > 0.10 | > 0.10 |
| hsa-miR-9-3p | 35.76 | **40.00** | **40.00** | 1 | *0.049\*\** | *0.047\** | **0.0047** | **0.0067** |
| hsa-miR-940 | 29.65 | 28.23 | 29.40 | 1 | 2.180 | 1.058 | > 0.10 | > 0.10 |
| hsa-miR-941 | 30.70 | 30.94 | 30.11 | 1 | 0.786 | 1.333 | > 0.10 | > 0.10 |
| hsa-miR-942-5p | 36.01 | 33.93 | 36.48 | 1 | 2.748 | 0.654 | > 0.10 | > 0.10 |
| hsa-miR-95-3p | 30.80 | 31.26 | 30.15 | 1 | *0.678\** | 1.398 | **0.0489** | > 0.10 |
| hsa-miR-9-5p | 33.45 | 34.89 | 34.89 | 1 | 0.343 | *0.328\** | 0.0675 | **0.0199** |
| hsa-miR-96-5p | 28.31 | 27.40 | 27.38 | 1 | 1.750 | *1.694\** | 0.0519 | **0.0463** |
| hsa-miR-98-5p | 29.92 | 29.71 | 29.16 | 1 | 1.076 | 1.500 | > 0.10 | 0.0973 |
| hsa-miR-99a-3p | 28.54 | 28.25 | 28.17 | 1 | 1.141 | 1.150 | > 0.10 | > 0.10 |
| hsa-miR-99a-5p | 21.16 | 21.25 | 21.08 | 1 | 0.874 | 0.937 | > 0.10 | > 0.10 |
| hsa-miR-99b-3p | 31.54 | 31.68 | 31.56 | 1 | 0.847 | 0.875 | > 0.10 | > 0.10 |
| hsa-miR-99b-5p | 25.81 | 25.83 | 25.76 | 1 | 0.921 | 0.918 | > 0.10 | > 0.10 |

The presence of 393 miRNAs (Cp value<38) was determined in the Nz samples (2 of them were not detected in OA - suggesting that they were specifically expressed in somatic cells from epididymis or testis- and 16 miRNAs were not expressed in either OA or NOA -suggesting a testicular specific expression-), whereas 4 miRNAs were detected in azoospermic samples but not in Nz samples, suggesting that they were minor miRNAs coming from other reproductive tissues different from epididymis and testis.

60 miRNAs in OA and/or NOA that presented significant differences in expression when compared with Nz controls were found (Table 5). In detail, regarding the OA-Nz comparison, 9 miRNAs were significantly overexpressed (fold-change increase range: 1.47-3.70; fold-change increase >2 n=4) and 42 underexpressed (fold-change decrease range: 1.47-1000; fold-change decrease >2 n=41). Among the miRNAs presenting the greatest fold-change decrease (>5Cp) we identified: miR-202-3p, miR-202-5p, miR-509-3-5p, miR-510-5p, miR-513c-5p, miR-514a-3p and miR-518e-3p. In the NOA samples, 8 miRNAs were significantly overexpressed (fold-change increase range: 1.24-9.45; fold-change increase >2 n=2) and 26 were underexpressed (fold-change decrease range: 1.13-1000; fold-change decrease >2 n=25) (Table 5) when compared with Nz controls.

**Table 5.-** Seminal exosome-derived miRNAs differentially expressed in OA and/or NOA compared with Nz individuals. Statistically increased miRNA expression levels are depicted in light grey; statistically decreased miRNA expression levels are depicted in dark grey, when compared with controls. * p<0.05; ** p<0.005. 1-6cX clusters in chromosome X. 1-2c19 clusters in chromosome 19.

| miRNA | Location | SP exosomal miRNA expression | | | Deduced miRNA origin from this work |
|---|---|---|---|---|---|
| | | Nz | OA | NOA | |
| **A. Underexpressed miRNAs** | | | | | |
| hsa-miR-202-3p | chr. 10 | 1 | 0.001** | 0.001** | Testis |
| hsa-miR-514a-3p$^{2cX}$ | chr. X | 1 | 0.001** | 0.001** | Testis |
| hsa-miR-202-5p | chr. 10 | 1 | 0.004** | 0.004** | Testis |
| hsa-miR-509-3-5p$^{1cX}$ | chr. X | 1 | 0.004** | 0.016* | Testis |
| hsa-miR-510-5p$^{2cX}$ | chr. X | 1 | 0.008** | 0.008** | Testis |
| hsa-miR-513c-5p$^{3cX}$ | chr. X | 1 | 0.010** | 0.009** | Testis |
| hsa-miR-518e-3p$^{1c19}$ | chr. 19 | 1 | 0.029** | 0.080* | Testis |

| | | | | | |
|---|---|---|---|---|---|
| hsa-miR-508-5p[1cX] | chr. X | 1 | 0.032** | 0.031** | Testis |
| hsa-miR-520h[2c19] | chr. 19 | 1 | 0.036** | 0.054* | Testis |
| hsa-miR-9-3p | chr. 1, 5, 15 | 1 | 0.049** | 0.047* | Testis |
| hsa-miR-506-3p[1cX] | chr. X | 1 | 0.050** | 0.047** | Testis |
| hsa-miR-383-5p | chr. 8 | 1 | 0.059** | 0.057** | Testis |
| hsa-miR-34c-5p | chr. 11 | 1 | 0.059** | 0.073* | Testis |
| hsa-miR-517c-3p[2c19] | chr. 19 | 1 | 0.069** | 0.091* | Testis |
| hsa-miR-873-5p | chr. 9 | 1 | 0.082** | 0.079** | Testis |
| hsa-miR-34b-5p | chr. 11 | 1 | 0.007* | 0.167* | Testis |
| hsa-miR-513a-3p[1cX] | chr. X | 1 | 0.036* | 0.034* | Testis |
| hsa-miR-521[1c19.2c19] | chr. 19 | 1 | 0.042* | 0.040* | Testis |
| hsa-miR-452-5p | chr. X | 1 | 0.063* | 0.148* | Testis |
| hsa-miR-122-5p | chr. 18 | 1 | 0.094* | 0.133* | Testis |
| hsa-miR-449a | chr. 5 | 1 | 0.237* | 0.205** | Testis |
| hsa-miR-499a-5p | chr. 20 | 1 | 0.343* | 0.315* | Testis |
| hsa-miR-455-5p | chr. 9 | 1 | 0.382* | 0.297* | Testis |
| hsa-miR-891b[6cX] | chr. X | 1 | 0.084* | 0.455 | Epididymis/Testis |
| hsa-miR-890[6cX] | chr. X | 1 | 0.090* | 0.363 | Epididymis/Testis |
| hsa-miR-34c-3p | chr. 11 | 1 | 0.093* | 0.382 | Epididymis/Testis |
| hsa-miR-891a-5p | chr. X | 1 | 0.097* | 0.448 | Epididymis/Testis |
| hsa-miR-888-5p[6cX] | chr. X | 1 | 0.098* | 0.391 | Epididymis/Testis |
| hsa-miR-124-3p | chr. 8, 20 | 1 | 0.101** | 0.481 | Epididymis/Testis |
| hsa-miR-892a[6cX] | chr. X | 1 | 0.107* | 0.544 | Epididymis/Testis |
| hsa-miR-551b-3p | chr. 3 | 1 | 0.139** | 0.246 | Epididymis/Testis |
| hsa-miR-424-5p | chr. X | 1 | 0.212* | 0.424 | Epididymis/Testis |
| hsa-miR-181b-5p | chr. 1, 9 | 1 | 0.246* | 0.362 | Epididymis/Testis |
| hsa-miR-31-3p | chr. 9 | 1 | 0.250* | 0.367 | Epididymis/Testis |
| hsa-miR-181a-5p | chr. 1, 9 | 1 | 0.292* | 0.459 | Epididymis/Testis |
| hsa-miR-31-5p | chr. 9 | 1 | 0.302* | 0.548 | Epididymis/Testis |
| hsa-miR-10b-3p | chr. 2 | 1 | 0.313* | 0.923 | Epididymis/Testis |
| hsa-miR-222-3p | chr. X | 1 | 0.323* | 0.570 | Epididymis/Testis |
| hsa-miR-455-3p | chr. 9 | 1 | 0.364* | 0.439 | Epididymis/Testis |
| hsa-miR-205-5p | chr. 1 | 1 | 0.367* | 0.734 | Epididymis/Testis |
| hsa-miR-182-3p | chr. 7 | 1 | 0.399* | 1.454 | Epididymis/Testis |
| hsa-miR-95-3p | chr. 4 | 1 | 0.678* | 1.398 | Epididymis/Testis |
| hsa-miR-9-5p | chr. 1, 5, 15 | 1 | 0.343 | 0.328* | |
| hsa-miR-132-5p | chr. 17 | 1 | 0.674 | 0.456* | |
| hsa-miR-203a | chr. 14 | 1 | 0.995 | 0.880* | |

**B. Overexpressed miRNAs**

| | | | | | |
|---|---|---|---|---|---|
| hsa-miR-363-3p[5cX] | chr. X | 1 | 1.474** | 1.241** | |
| hsa-miR-365a-3p | chr. 16 | 1 | 1.689* | 1.394* | |

| hsa-miR-29a-3p | chr. 7 | 1 | 1.614* | 1.288 |
| hsa-miR-296-5p | chr. 20 | 1 | 3.705* | 2.400 |
| hsa-miR-23b-5p | chr. 9 | 1 | 2.590* | 1.498 |
| hsa-miR-21-3p | chr. 17 | 1 | 2.202* | 1.485 |
| hsa-miR-193a-3p | chr. 17 | 1 | 2.879* | 1.783 |
| hsa-miR-29c-3p | chr. 1 | 1 | 1.687* | 1.415 |
| hsa-miR-361-3p | chr. X | 1 | 1.741* | 1.575 |
| hsa-miR-550a-5p | chr. 7 | 1 | 2.465 | 9.455* |
| hsa-miR-423-5p | chr. 17 | 1 | 1.129 | 1.485* |
| hsa-let-7f-1-3p | chr. 9 | 1 | 1.093 | 1.808* |
| hsa-miR-153-3p | chr. 2, 7 | 1 | 1.667 | 2.251* |
| hsa-miR-196b-3p | chr. 7 | 1 | 1.123 | 1.451* |
| hsa-miR-96-5p | chr. 7 | 1 | 1.750 | 1.694* |

Twenty-five of the differentially expressed miRNAs were shared among OA and NOA groups, either down-(n=23) or up-regulated (n=2), when compared with Nz controls (Table 2). Remarkably, among the shared down-regulated miRNAs in azoospermia, there was no expression value (Cp value>38) for miR-202-3p, miR-202-5p, miR-383-5p, miR-506-3p, miR-508-5p, miR-510-5p, miR-513a-3p, miR-513c-5p, miR-514a-3p, miR-518e-3p, miR-520h, miR-521, miR-873-5p and miR-9-3p (see Table 4). Those exosomal miRNAs that presented statistically lower levels in both OA and NOA men were candidates for having preferentially derived from the testicular cells associated with the presence of germline (n=23), whereas the 19 miRNAs found to be downregulated in OA but not in NOA samples are suggested to be preferentially secreted by somatic cells from the testis and/or the epididymis (Table 5). Interestingly, among the latter group of miRNAs we found several miRNAs described to be epididymis-specific such as the X-linked miR-888 cluster.

## EXAMPLE 3.- Exosome miRNA levels in SP identify azoospermia with different origin

Subsequently, the miRNA expression profile of SP exosomes between NOA and OA-V samples was compared. 12 miRNAs (Figure 2) presented significant differences in expression between groups with potential implications for diagnosis were found. According to this profiling outcome, several miRNAs were validated as candidate biomarkers of the origin of azoospermia.

First, 5 miRNAs (miR-182-3p, miR-205-5p, miR-31-5p, miR-539-5p, miR-941) were selected for validation in a larger number of individuals based on the following criteria: we selected those miRNAs that presented $\geq$1.7-fold difference in expression between groups and Cp value <34 in any of the azoospermic groups (Figure 2). We included in the validation stage of the study 3 additional miRNAs, such as miR-122-5p, miR-34c-5p, miR-449a, that we found differentially expressed in SP exosomes between azoospermic and Nz individuals (Table 5) and were previously described as being preferentially produced from the germline of the testis: miR-34c-5p, miR-449a are localized to spermatocytes and spermatids and miR-122-5p is enriched in late-stage male germ-cells. Additionally, the cellular content of all these three miRNAs were found to be decreased in developing germ-cells of SpF testis and in mature sperm from mild SpF patients; and the combination of the expression values of these three miRNAs in testicular biopsies is able to predict the availability of sperm in the biopsy for assisted reproduction treatment. PiRNAs are sncRNAs predominantly expressed in the germline. Thus, the piRNA piR-58527 (piRNABank accession DQ591415) was additionally included in the validation study as it is specifically expressed in the postmeotic germ cells.

In order to determine the expression level of each miRNA in the different organs of the reproductive tract, the expression of the 8 miRNAs was first tested in testis, epididymis, prostate, seminal plasma and lymphocytes, the latter as external control cells (Figure 3). We could corroborate the preferential testicular expression of miR-122-5p, miR-34c-5p and miR-449a, whereas miR-205-5p is preferentially expressed in epididymis and prostate. The miR-182-3p, miR-31-5p, miR-539-5p and miR-941 are expressed in the three reproductive organs (testis, epididymis and prostate).

Therefore, these particular 8 miRNAs and 1 piRNA were individually reanalyzed in a subsequent set of semen samples (7 Nz samples, 14 NOA samples, 10 OA samples including 5 OA-V samples and 5 OA-N samples, and 3 oligozoospermic samples) by RT-qPCR (Figure 1). First, the inventors' results showed that the expression values of miR-122-5p ($p$<0.0001), piR-58527 ($p$<0.0001), and miR-539-5p ($p$=0.034) were statistically different between azoospermic and normozoospermic individuals (Figure 4, significant differences for this comparison not shown). Interestingly, the expression values of miR-122-5p was statistically correlated with the concentration of sperm in semen (r=0.616, $p<0.0001$).

Furthermore, when the NOA and OA samples were compared, the inventors' results show that the tendencies among the 9 miRNAs/piRNA analyzed were conserved among the two different approaches (miRNA qPCR array and RT-qPCR), although only two of them (miR-205-5p, miR-31-5p; $p<0.004$) as well as piR-58527 ($p=0.031$) were found to be statistically different between both groups of azoospermia in the RT-qPCR validation analysis (Figure 4, significant differences for this comparison not shown).

**Table 6.-** ROC analysis showing the predictive efficiency of miRNA variables for distinguishing NOA from OA samples at the validation stage.

| Variable | AUC | Variable | AUC |
|---|---|---|---|
| miR-182-3p | 0.741 | piR-58527 | 0.744 |
| miR-205-5p | 0.838 | miR-122-5p | 0.723 |
| miR-31-5p | 0.963 | miR-34c-5p | 0.750 |
| miR-539-5p | 0.650 | miR-449a | 0.348 |
| miR-941 | 0.603 | | |

The expression values of these 3 small non-coding RNAs resulted in good predictive accuracy [miR-205-5p (AUC 0.843, $p=0.005$); miR-31-5p (AUC: 0.957; $p<0.0001$); piR-58527 (AUC: 0.764; $p=0.030$)] after ROC curve analysis, suggesting they have a potential use as indicators of the origin of the azoospermia. As a comparison, the ROC curve analysis of blood FSH levels was also determined (AUC 0.850, $p=0.004$). To determine if a multiplex model could improve performance over single biomarkers for discriminating NOA from OA samples, the 3 previously selected miRNAs were analyzed in a multivariate logistic regression analysis. Interestingly, this analysis resulted in a model that only included the miR-31-5p expression values. The sensitivity and the specificity for predicting the origin of azoospermia were 92.9 and 90%, respectively (Figure 5). Strikingly, an increased value of sensitivity and specificity (100%) was obtained when (FSH + miR-31-5p) values were included in the model.

Interestingly, when the OA sample group was divided into OA-N and OA-V sub-phenotypes, three miRNAs [miR-205-5p ($p=0.010$); miR-31-5p ($p=0.001$); and miR-539-5p ($p=0.026$)] were differentially expressed between OA-N and NOA samples; whereas miR-31-5p and miR-941 ($p<0.003$) were downregulated in OA-V compared with NOA samples. This observation suggests that the profile of exosomal miRNA in semen is different in congenital obstruction of the genital tract from that obtained from the vasectomy procedure, and probably depends on the level at which the obstruction of the genital tract is produced. We then specifically focused on the differences between naturally-occurring OA-N and NOA samples. The expression values of the 3 differentially expressed miRNAs resulted in good predictive accuracy [miR-205-5p (AUC: 0.886, $p=0.012$); miR-31-5p (AUC: 0.957; $p=0.003$); miR-539-5p (AUC: 0.843; $p=0.026$)] after ROC curve analysis, suggesting they have a potential use as indicators of the origin of the naturally-occurring azoospermia, as well as FSH (AUC: 0.843, $p=0.026$).

When a multivariate regression analysis was performed, it resulted in a model that, again, only included the miR-31-5p expression values. The sensitivity and the specificity for predicting the origin of naturally occurring azoospermia were 92.9 and 80%, respectively, confirming that exosomal miR-31-5p expression values in semen samples could be useful to predict the presence of a conserved spermatogenesis in the testes (OA). Once again, an increased value of sensitivity and specificity (100%) was obtained when (FSH + miR-31-5p) were included in the predictive model.

The binary logistic regression model provides the following estimation of the logit function:

$$\text{Logit(p)} = B0 + B1X1 + B2X2 + \ldots \quad \text{(Formula I)}.$$

In particular, in the present analysis, p=P (presence of sperm in the testis), Logit(p)=log(p/(1-p))=log(Odds), B=log OR and Xn= the expression value of the selected miRNA and FSH. Therefore, when this estimated model as a prediction model, was used with the standard classification cutoff of 0.5, individuals with a positive Logit function estimation would be classified as "adequate presence of sperm in the testis: obstructive azoospermia" and individuals with negative Logit function estimation would be classified as "no adequate presence of sperm in the testis: non-obstructive azoospermia".

**EXAMPLE 4.- Exosome miRNA levels in SP and the presence of intratubular mature germ cells in spermatogenic disorders**

In order to assess if miRNA expression and histological parameters from a biopsy with secretory azoospermia were associated and to know the physiological relevance and/or the potential implications for reproductive treatments, miRNA profile of 4 NOA individuals (no. 18-21) with no spermatozoa in the testicular biopsy (Sp -) and 8 NOA samples (no. 10-17) with presence of intra-testicular sperm (Sp +), the latter including the samples from cryptozoospermic individuals and those samples from individuals with a positive TESE value (>0.01x10$^6$ sperm/ml) (Table 1), were compared. The TESE value is defined as the number of spermatozoa that was obtained directly after processing 100 mg of biopsy in 1mL of medium.

Multivariate binary logistic regressions were used for selection of the optimal combination of miRNAs in SP exosomes associated with the presence of residual spermatogenesis in the testes as a predictive tool. A backward stepwise (conditional) method was used to drop insignificant terms. The multivariate regression model included the 5 miRNAs found to significantly distinguish NOA from OA-V samples in the miRNA profiling phase and the 3 germline specific miRNAs and 1 piRNA.

When the multivariate regression analysis was performed, it resulted in a model that included the miR-539-5p and the miR-941 expression values.

The binary logistic regression model provides the following estimation of the logit function:

$$\text{Logit}(p) = B0 + B1X1 + B2X2 + \ldots \quad \text{(Formula I).}$$

In particular, Logit(p)= B0 + B1X1 +B2X2, wherein:

p=P (presence of sperm in the testes),
Logit(p)=log(p/(1-p))=log(Odds),
B=log OR, and
Xn= the expression value of the selected miRNAs, i.e. X1: miR-539-5p, X2: miR-941. Thus, for the samples used in the present assay, B variables were as follow: B0= log OR constant; B1= log OR miR-539-5p, B2 log OR miR-941.

Therefore, using this estimated model as a prediction model, with the standard classification cutoff of 0.5, individuals with a positive Logit function estimation would be classified as "residual spermatogenesis" (presence of sperm in the testes) and individuals with a negative Logit function estimation would be classified as "no residual spermatogenesis" (absence of sperm in the testes).

Interestingly, the sensitivity and the specificity for predicting the presence of spermatozoa in a testicular biopsy were both 100%. When FSH was incorporated in the regression analysis, the model included miR-539-5p, miR-941 and FSH values resulting in true positive and negative rates for predicting residual spermatogenesis in the testes of again 100%.

Literature

Cooper et al., World Health Organization reference values for human semen characteristics. Hum Reprod Update 2010; 16: 231-245.

Crescitelli et al., Distinct RNA profiles in subpopulations of extracellular vesicles: apoptotic bodies, microvesicles and exosomes. J Extracell Vesicles 2013; 2: 10.3402/jev.v2i0.20677.

Li et al., Cell-free seminal mRNA and microRNA exist in different forms. PLoS One 2012; 7: e34566.

**Claims**

1. An *in vitro* method for predicting the presence of sperm in the testes of a subject, comprising the analysis of the expression profile of a predetermined set of miRNAs in a biological sample taken from said subject, wherein said subject has non-obstructive azoospermia and wherein said predetermined set of miRNAs comprises miR-539-5p and miR-941.

2. The *in vitro* method according to claim 1, wherein the subject is an azoospermic subject who is identified as having non-obstructive azoospermia by analysing a predetermined set of miRNAs in a biological sample taken from said subject, wherein the predetermined set of miRNA comprises one or more miRNAs selected from the group consisting of miR-31-5p, miR-539-5p, miR-941, miR-205-5p, miR-182-3p, miR-192-3p, miR-19a-3p, miR-200a-3p, miR-23b-5p, miR-363-3p, miR-451a, miR-934 and combination thereof, more preferably miR-31-5p and/or miR-205-5p.

3. An *in vitro* method for determining the origin of azoospermia, comprising the analysis of the expression profile of a predetermined set of miRNAs in a biological sample taken from an azoospermic subject, wherein said predetermined set of miRNAs comprises one or more miRNAs selected from the group consisting of miR-31-5p, miR-539-5p, miR-941, miR-205-5p, miR-182-3p, miR-192-3p, miR-19a-3p, miR-200a-3p, miR-23b-5p, miR-363-3p, miR-451a, miR-934 and combination thereof, preferably miR-31-5p or miR-205-5p or miR-539-5p or combinations thereof.

4. The *in vitro* method according to claim 2 or 3, wherein the predetermined set of miRNAs comprises, preferably consists of, miR-31-5p.

5. An *in vitro* method for predicting the presence of sperm in the testes of a subject, comprising the analysis of the expression profile of a predetermined set of miRNAs in a biological sample taken from said subject, wherein said subject has azoospermia and wherein said predetermined set of miRNAs comprises miR-31-5p and miR-941.

6. The *in vitro* method according to any one of claims 1-5, further comprising the analysis of the FSH value in a blood sample taken from the subject.

7. A set of miRNAs for use in predicting the presence of sperm in the testes of a subject with non-obstructive azoospermia, wherein the set of miRNAs comprises miR-539-5p and miR-941, and is isolated from a biological sample of said subject.

8. A set of miRNAs for use in predicting the presence of sperm in the testes of a subject with azoospermia, wherein the set of miRNAs comprises miR-31-5p and miR-941, and optionally miR-539-5p, and is isolated from a biological sample of said subject.

9. A set of miRNAs for use in discriminating between obstructive and non-obstructive azoospermia, wherein the set of miRNAs is isolated from a biological sample of said subject and comprises one or more miRNAs selected from the group consisting of miR-31-5p, miR-539-5p, miR-941, miR-205-5p, miR-182-3p, miR-192-3p, miR-19a-3p, miR-200a-3p, miR-23b-5p, miR-363-3p, miR-451a, miR-934 and combination thereof, preferably miR-31-5p or miR-205-5p or miR-539-5p or combinations thereof, and more preferably miR-31-5p.

10. The *in vitro* method according to any one of claims 1-6, or the set of miRNAs for use according to any one of claims 7-9, wherein the biological sample is semen or seminal plasma, and preferably the biological sample comprises isolated extracellular vesicles, preferably isolated exosomes, from semen or seminal plasma.

11. Kit for carrying out the method as defined in claim 1, comprising:

a) appropriate means to determining the expression profile of a predetermined set of miRNAs comprising, preferably consisting of, miR-539-5p and miR-941;
and a suitable packaging.

12. Kit for carrying out the method as defined in claim 3, comprising:

a) appropriate means to determining the expression profile of a predetermined set of miRNAs comprising one or more miRNAs selected from the group consisting of miR-31-5p, miR-205-5p, miR-182-3p, miR-192-3p, miR-19a-3p, miR-200a-3p, miR-23b-5p, miR-363-3p, miR-451a, miR-934 and combination thereof, more preferably miR-31-5p, and/or miR-205-5p;
and a suitable packaging.

13. Kit for carrying out the method as defined in claim 5, comprising:

a) appropriate means to determining the expression profile of a predetermined set of miRNAs comprising, preferably consisting of, miR-31-5p and miR-941;
and a suitable packaging.

14. Use of a kit according to claim 11 or 13, for predicting the presence of sperm in the testes of a subject with non-obstructive azoospermia or azoospermia, respectively, or use of a kit according to claim 12, for discriminating between non-obstructive azoospermia and obstructive azoospermia.

15. The method according to any one of claims 1-6 and 10, the set of miRNA for use according to any one of claims 7-10, the kit according to any one of claims 11-13 or the use of a kit according to claim 14, wherein miR-539-5p, miR-941, miR-31-5p, miR-205-5p, miR-182-3p, miR-192-3p, miR-19a-3p, miR-200a-3p, miR-23b-5p, miR-363-3p, miR-451a and miR-934 are hsa-miR-539-5p (SEQ ID NO 1), hsa-miR-941 (SEQ ID NO 2), hsa-miR-31-5p (SEQ ID NO 3), hsa-miR-205-5p (SEQ ID NO 4), hsa-miR-182-3p (SEQ ID NO 5), hsa-miR-192-3p (SEQ ID NO 6), hsa-miR-19a-3p (SEQ ID NO 7), hsa-miR-200a-3p (SEQ ID NO 8), hsa-miR-23b-5p (SEQ ID NO 9), hsa-miR-363-3p (SEQ ID NO 10), hsa-miR-451a (SEQ ID NO 11) and hsa-miR-934 (SEQ ID NO 12), respectively.

**FIGURE 1**

FIGURE 2

FIGURE 3

**FIGURE 4**

**FIGURE 4, cont.**

**E**

**F**

# FIGURE 4, cont.

**G**

**H**

# FIGURE 4, cont.

I

**FIGURE 4, cont.**

**FIGURE 5**

Europäisches
Patentamt

European
Patent Office

Office européen
des brevets

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LIAN JIE ET AL: "Altered microRNA expression in patients with non-obstructive azoospermia", REPRODUCTIVE BIOLOGY AND ENDOCRINOLOGY, BIOMED CENTRAL LTD, GB, vol. 7, no. 1, 11 February 2009 (2009-02-11), page 13, XP021050451, ISSN: 1477-7827, DOI: 10.1186/1477-7827-7-13 * page 2, column 1; page 4, figure 1 * | 7-10,15 | INV. C12Q1/6883 |
| X | XUAN ZHUANG ET AL: "Integrated miRNA and mRNA expression profiling to identify mRNA targets of dysregulated miRNAs in non-obstructive azoospermia", SCIENTIFIC REPORTS, vol. 5, no. 1, 28 January 2015 (2015-01-28), XP055480279, DOI: 10.1038/srep07922 * page 2, "Methods"; page 4, figure 2 * | 7-10,15 | |
| X | WO 2014/180981 A1 (INSERM INST NAT DE LA SANTÉ ET DE LA RECH MÉDICALE [FR]; UNIV NICE SOP) 13 November 2014 (2014-11-13) * page 11, "Kits" * | 11-13 | TECHNICAL FIELDS SEARCHED (IPC) C12Q |

-/--

The present search report has been drawn up for all claims

| Place of search | Date of completion of the search | Examiner |
|---|---|---|
| Munich | 1 June 2018 | Hennard, Christophe |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 1 of 2

**EUROPEAN SEARCH REPORT**

Application Number

EP 18 38 2194

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XAVIER MUÑOZ ET AL: "Altered miRNA Signature of Developing Germ-cells in Infertile Patients Relates to the Severity of Spermatogenic Failure and Persists in Spermatozoa", SCIENTIFIC REPORTS, vol. 5, no. 1, 9 December 2015 (2015-12-09), XP055480282, DOI: 10.1038/srep17991 * abstract; page 10, "Material and methods"; bridging paragraph of pages 9-10 * | 1-15 | |
| X,P | MARIA BARCELÓ ET AL: "Exosomal microRNAs in seminal plasma are markers of the origin of azoospermia and can predict the presence of sperm in testicular tissue", HUMAN REPRODUCTION, vol. 33, no. 6, 4 April 2018 (2018-04-04), pages 1087-1098, XP055480289, GB ISSN: 0268-1161, DOI: 10.1093/humrep/dey072 * Abstract; page 1089; tables * | 1-15 | |

TECHNICAL FIELDS SEARCHED (IPC)

The present search report has been drawn up for all claims

| Place of search | Date of completion of the search | Examiner |
|---|---|---|
| Munich | 1 June 2018 | Hennard, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 38 2194

This annex lists the patent family members relating to the patent documents cited in the above-mentioned European search report.
The members are as contained in the European Patent Office EDP file on
The European Patent Office is in no way liable for these particulars which are merely given for the purpose of information.

01-06-2018

| Patent document cited in search report | Publication date | Patent family member(s) | Publication date |
|---|---|---|---|
| WO 2014180981 A1 | 13-11-2014 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011080315 A1
- EP 2598657 B1
- CN 106337088 A1

**Non-patent literature cited in the description**

- **COOPER et al.** World Health Organization reference values for human semen characteristics. *Hum Reprod Update,* 2010, vol. 16, 231-245
- **CRESCITELLI et al.** Distinct RNA profiles in subpopulations of extracellular vesicles: apoptotic bodies, microvesicles and exosomes. *J Extracell Vesicles,* 2013, vol. 2
- **LI et al.** Cell-free seminal mRNA and microRNA exist in different forms. *PLoS One,* 2012, vol. 7, e34566

SEQUENCE LISTING

<110> Institut d'Investigació Biomedica de Bellvitge (IDIBELL)
Fundació Puigvert

<120> METHODS AND MARKERS FOR AZOOSPERMIA CHARACTERISATION

<130> EP20171848

<160> 14

<170> PatentIn version 3.5

<210> 1
<211> 22
<212> RNA
<213> Homo sapiens

<400> 1
ggagaaauua uccuuggugu gu                                              22


<210> 2
<211> 23
<212> RNA
<213> Homo sapiens

<400> 2
cacccggcug ugugcacaug ugc                                             23


<210> 3
<211> 21
<212> RNA
<213> Homo sapiens

<400> 3
aggcaagaug cuggcauagc u                                               21


<210> 4
<211> 22
<212> RNA
<213> Homo sapiens

<400> 4
uccuucauuc caccggaguc ug                                              22


<210> 5
<211> 21
<212> RNA
<213> Homo sapiens

<400> 5
ugguucuaga cuugccaacu a                                               21


<210> 6
<211> 22
<212> RNA
<213> Homo sapiens

```
<400>  6
cugccaauuc cauaggucac ag                                                22


<210>  7
<211>  23
<212>  RNA
<213>  Homo sapiens

<400>  7
ugugcaaauc uaugcaaaac uga                                               23


<210>  8
<211>  22
<212>  RNA
<213>  Homo sapiens

<400>  8
uaacacuguc ugguaacgau gu                                                22


<210>  9
<211>  22
<212>  RNA
<213>  Homo sapiens

<400>  9
uggguuccug gcaugcugau uu                                                22


<210>  10
<211>  22
<212>  RNA
<213>  Homo sapiens

<400>  10
aauugcacgg uauccaucug ua                                                22


<210>  11
<211>  22
<212>  RNA
<213>  Homo sapiens

<400>  11
aaaccguuac cauuacugag uu                                                22


<210>  12
<211>  22
<212>  RNA
<213>  Homo sapiens

<400>  12
ugucuacuac uggagacacu gg                                                22


<210>  13
<211>  22
<212>  RNA
<213>  Homo sapiens
```

```
<400>   13
uguaaacauc cccgacugga ag                                               22


<210>   14
<211>   22
<212>   RNA
<213>   Homo sapiens

<400>   14
cuuucagucg gauguuuaca gc                                               22
```